(19) Europäisches Patentamt
European Patent Office
Office européen des brevets

(11) **EP 4 328 578 B1**

(12) **EUROPÄISCHE PATENTSCHRIFT**

(45) Veröffentlichungstag und Bekanntmachung des Hinweises auf die Patenterteilung:
**15.01.2025   Patentblatt 2025/03**

(21) Anmeldenummer: **23192677.5**

(22) Anmeldetag: **22.08.2023**

(51) Internationale Patentklassifikation (IPC):
**G01N 29/02** *(2006.01)*

(52) Gemeinsame Patentklassifikation (CPC):
**G01N 29/022;** G01N 2291/0255; G01N 2291/0256; G01N 2291/0427

(54) **DIGITALE SENSORVORRICHTUNG ZUR DETEKTION VON ANALYTEN IN EINER PROBE**

DIGITAL SENSOR DEVICE FOR DETECTING ANALYTES IN A SAMPLE

DISPOSITIF DE CAPTEUR NUMÉRIQUE POUR DÉTECTER DES ANALYTES DANS UN ÉCHANTILLON

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität:  **22.08.2022   DE 102022121187**

(43) Veröffentlichungstag der Anmeldung:
**28.02.2024   Patentblatt 2024/09**

(73) Patentinhaber: **digid GmbH**
**55129 Mainz (DE)**

(72) Erfinder:
• **Köhler, Malte**
**55129 Mainz (DE)**

• **Kloppstech, Konstantin**
**55129 Mainz (DE)**
• **von Gersdorff, Constantin**
**55129 Mainz (DE)**
• **Könne, Nils**
**55129 Mainz (DE)**

(74) Vertreter: **Nordmeyer, Philipp Werner**
**df-mp Dörries Frank-Molnia & Pohlman**
**Patentanwälte Rechtsanwälte PartG mbB**
**Theatinerstraße 16**
**80333 München (DE)**

(56) Entgegenhaltungen:
**US-A1- 2004 152 211     US-B1- 6 575 020**

**Beschreibung**

Technisches Gebiet

Die vorliegende Erfindung betrifft eine Sensorvorrichtung zur Detektion eines oder mehrerer Analyten in einer Probe, um eine qualitative und/oder quantitative Aussage über das Vorliegen und/oder eine Konzentration des oder der Analyten in der Probe abzuleiten.

Stand der Technik

Eine hochsensitive Methode zur Detektion und Analyse von Analyten in einer Probe besteht darin, eine durch einen Analyten induzierte mikroskopische Verformung eines miniaturisierten Federelements, eines so genannten Cantilevers, zu verwenden, um die Präsenz eines Analyten in einer Probe zu messen. Eine spezifisch durch einen Analyten hervorgerufene Verformung wird erreicht, indem der Cantilever einseitig mit einer Rezeptorschicht versehen wird, welche Bindemoleküle aufweist, die spezifisch an einen bestimmten zu untersuchenden Analyten bindet und dadurch eine einseitig veränderte Oberflächenspannung des Cantilevers hervorruft. Dabei wird die Verformung des Cantilevers insbesondere durch einen auf dem Cantilever aufgebrachten elektrischen Wandler bzw. Transducer in eine elektrisch detektierte Größe gewandelt, um die Präsenz des Analyten quantitativ zu messen. Ein solches Federelement wird beispielsweise in der DE 10 2020 107 918 A1 beschrieben.

Die WO 2007/088018 A1 schlägt Federelemente zur Verwendung in Biosensoren wie beispielsweise zur DNA-Analyse vor.

Die Verformung von Cantilevern durch unterschiedliche Oberflächenspannungen ist beispielsweise in Rasmussen, P. A., Hansen, O., & Boisen, A. (2005) "Cantilever surface stress sensors with single-crystalline silicon piezoresistors" Applied Physics Letters, 86(20), 203502. https://doi.org/10.1063/1.1900299 beschrieben.

Die US 6 575 020 B1 beschreibt integrierte Mikrocantilever in Mikroflüssigkeits-Handhabungssystemen.

Darstellung der Erfindung

Ausgehend von dem bekannten Stand der Technik ist es eine Aufgabe der vorliegenden Erfindung, eine verbesserte Sensorvorrichtung zur Detektion eines Analyten in einer Probe bereitzustellen.

Die Aufgabe wird durch eine Sensorvorrichtung zur Detektion eines Analyten in Probe mit den Merkmalen des Anspruchs 1 gelöst. Vorteilhafte Weiterbildungen ergeben sich aus den Unteransprüchen, der Beschreibung und den Figuren.

Entsprechend wird ein Sensor zur Umwandlung chemischer und/oder biochemischer Information eines Analyten in ein elektrisches Signal in einer Probe vorgeschlagen. Der Sensor umfasst einen Testcantilever, der eine Basis und einen verformbaren Teil aufweist, wobei mindestens auf dem verformbaren Teil eine Rezeptorschicht zur selektiven Aufnahme des Analyten aufgebracht ist, wobei der Testcantilever eine Test-Wandlerschicht aufweist, die auf der Basis einen passiven Testtransducer und auf dem verformbaren Teil einen aktiven Testtransducer umfasst, und wobei zwischen der Test-Wandlerschicht und der Rezeptorschicht eine Test-Funktionsschicht angeordnet ist. Weiterhin umfasst der Sensor einen Referenzcantilever, der eine Basis und einen verformbaren Teil aufweist, wobei auf dem verformbaren Teil eine Referenzschicht zur selektiven Nichtaufnahme des Analyten aufgebracht ist, wobei der Referenzcantilever eine Referenz-Wandlerschicht aufweist, die auf der Basis einen passiven Referenztransducer und auf dem verformbaren Teil einen aktiven Referenztransducer umfasst, und wobei zwischen der Referenz-Wandlerschicht und der Referenzschicht eine Referenz-Funktionsschicht angeordnet ist. Dabei sind die aktiven und passiven Referenztransducer sowie die aktiven und passiven Testtransducer dazu ausgebildet und eingerichtet, ein dem Vorkommen und/oder der Konzentration und/oder der Menge des Analyten oder der Analyten in der Probe entsprechendes elektrisches Signal auszugeben.

Die Basis des Testcantilevers und/oder die Basis des Referenzcantilevers können als starre Basis ausgebildet sein. Unter einer starren Basis wird dabei verstanden, dass eine Verformung des jeweiligen Cantilevers, also des Testcantilevers und/oder des Referenzcantilevers, gegenüber dem verformbaren Teil des jeweiligen Cantilevers nicht oder im Wesentlichen nicht stattfindet. Die starre Basis ist dabei beispielsweise mit einem Substrat verbunden, durch ein Substrat unterstützt oder aus dem Substrat herausgearbeitet. Der verformbare Teil des Testcantilevers und/oder des Referenzcantilevers ist hingegen nicht von dem Substrat unterstützt, sondern vielmehr über eine Kante des Substrats herausragend und entsprechend frei ausgebildet.

Der verformbare Teil des Testcantilevers und/oder des Referenzcantilevers kann beispielsweise auslenkbar ausgebildet sein. Dabei kann eine Auslenkung des jeweiligen Cantilevers beispielsweise um eine in einem Übergangsbereich zwischen Basis und auslenkbarem Bereich ausgeformte Biegekante herum erreicht werden. Die Biegekante ist hierbei beispielsweise die Kante des Substrats entlang derer der Cantilever in die Basis und den verformbaren Teil unterteilt wird.

Die Verformung des jeweiligen Cantilevers in seinem verformbaren Teil ist aber nicht begrenzt auf eine anhebende oder absenkende Verformung, auch der Cantilever kann in sich selbst verformt werden, beispielsweise aufgewölbt oder gewellt oder verzerrt.

Eine Probe bezeichnet hierbei eine beschränkte Menge eines Stoffes, die einer größeren Menge des Stoffes, etwa aus einem Reservoir, entnommen wurde, wobei die Zusammensetzung der Probe repräsentativ für die Zusammensetzung des Stoffs in dem Reservoir ist und dementsprechend aus dem Stoffvorkommen und Stoffzusammensetzungen der Probe auf das entsprechende Vorkommen im Reservoir geschlossen werden kann.

Beispielsweise kann eine Probe eine Speichelprobe, oder eine Blutprobe, oder Lymphe, oder Urin, oder Schweiß, oder Intergewebsflüssigkeit, oder ein Abstrich sein, insbesondere ein Rachenabstrich oder ein Nasenabstrich oder ein Nebenhöhlenabstrich sein, oder entnommenes Gewebe sein. Eine Probe umfasst insbesondere jegliche Art von biologischer Probe, also insbesondere auch Proben von Tieren.

Eine Probe kann auch eine nichtbiologische Probe, beispielsweise eine Probe eines chemischen Stoffs, sein.

Ein Analyt ist hierbei der Stoff, dessen Vorliegen in der Probe qualitativ und/oder quantitativ nachgewiesen werden soll beziehungsweise mit dem Sensor detektiert werden soll. Der Analyt kann insbesondere unmittelbar in der Probe vorhanden sein, oder in der Probe gelöst sein oder der Probe oder einem Teil der Probe, insbesondere einem Probenpartikel, anhaften. Der Analyt kann mit der Probe auch eine chemische, biologische und/oder physikalische Wechselwirkung eingehen, sodass der Analyt lediglich indirekt über eine entsprechende Wechselwirkung detektierbar ist.

Insbesondere kann eine Probenform in eine weitere Probenform überführt werden, sodass der Analyt, beziehungsweise dessen Vorkommen in einer einfacher und sicheren Art und Weise detektiert werden kann. Beispielsweise kann ein Abstrich in einer Flüssigkeit gelöst werden, sodass der in der Flüssigkeit gelöste Abstrich dann die eigentliche Probe ist, welche auf den Analyten hin untersucht wird.

Der Analyt in der Probe kann auch chemisch vorbehandelt werden, beispielsweise indem - sofern der Analyt ein Virus ist - die Virushülle aufgeschlossen wird, um an Nucleocapsid-Antigene zu gelangen. Weiterhin kann der Analyt durch eine solche Vorbehandlung auch "gelabeled" werden, um das Messsignal zu verstärken. Hierzu können z.B. konjugierte Antikörper an Antigene des Analyten binden, um auf dem Cantileversystem eine möglichst große Verformung zu erzeugen.

Die Probe enthält dann die chemische Information und/oder biochemische Information über den Analyten. Die chemische Information kann beispielsweise die Art des Analyten, die Konzentration des Analyten, das Vorkommen des Analyten, das Gewicht des Analyten, die Reaktivität des Analyten, die Dichte des Analyten usw. umfassen. Die biochemische Information umfasst dieselben Eigenschaften wie die chemische Information, jedoch können diese Stoffe beispielsweise durch biologische Prozesse entstehen. Insbesondere spricht man von biochemischer Information, wenn der Analyt einen besonderen Einfluss auf den biologischen Kreislauf, beispielsweise den Stoffwechsel oder auf das Immunsystem hat.

Der Testcantilever und der Referenzcantilever umfasst jeweils eine Test-Wandlerschicht und eine Referenz-Wandlerschicht, in welche die passiven und aktiven Testtranducer und Referenztransducer eingebettet sind. Die Test-Wandlerschicht und Referenz-Wandlerschicht sind vornehmlich dazu eingerichtet, die passiven und/oder aktiven Testtransducer sowie die passiven und/oder aktiven Referenztransducer elektrisch zu kontaktieren. Die Wandlerschichten können auch aus mehreren verschiedenen Schichten und Materialien bestehen. Weiterhin können die Test-Wandlerschicht und die Referenz-Wandlerschicht weitere Funktionen umfassen, welche später im Detail diskutiert werden.

Bevorzugt ist innerhalb der Test-Wandlerschicht auf der Basis des Testcantilevers ein passiver Testtransducer angeordnet und auf dem verformbaren Teil des Testcantilevers ein aktiver Testtransducer angeordnet, während innerhalb der Referenz-Wandlerschicht auf der Basis des Refenzcantilevers ein passiver Referenztransducer angeordnet und auf dem verformbaren Teil des Referenzcantilevers ein aktiver Referenztransducer angeordnet ist, wobei die aktiven und passiven Referenztransducer und die aktiven und passiven Testtransducer dazu ausgebildet und eingerichtet sind, ein dem Vorkommen und/oder Konzentration und/oder der Menge des Analyten in der Probe entsprechendes elektrisches Signal auszugeben.

Die chemische und/oder biochemische Information wird in ein elektrisches Signal umgewandelt. Dies kann bedeuten, dass von der chemischen Zusammensetzung des Analyten ein elektrisches Signal verändert oder aufgebaut werden kann. Dies kann beispielsweise die Leitfähigkeit eines Schaltkreises betreffen. Beispielsweise kann eine erste biochemische Information vorliegen, wenn der Schaltkreis leitet, und eine zweite biochemische Information vorliegen, wenn der Schaltkreis nicht leitet oder eine verminderte Leitfähigkeit aufweist.

Neben der direkten Zugänglichkeit der Information, wie beispielsweise über die Leitfähigkeit, ist es aber auch möglich, über einen physikalischen und oder chemischen Prozess und/oder eine Wechselwirkung auf die biochemische Information zu schließen.

Zu diesem Zweck umfasst der vorgeschlagene Sensor einen Referenz- und einen Testcantilever. Ein Cantilever ist hierbei ein Federelement, welches eine Basis und einen verformbaren Teil aufweist. Die Basis ist dementsprechend ein unbeweglicher Teil des Cantilevers, der insbesondere ortsfest auf einem Substrat angeordnet ist. Der verformbare Teil des Cantilevers ist an der Basis angeordnet und ragt über die Basis hinaus.

Insbesondere können die Basis und der Cantilever einteilig miteinander ausgebildet sein. Mit anderen Worten ist der verformbare Teil des Cantilevers einseitig an der Basis aufgehängt. In dem der verformbare Teil über das Substrat hinaus ragt, lässt sich der verformbare Teil des Cantilevers verbiegen, auslenken und dehnen. Die räumliche Grenze, ab der der Cantilever biegbar ist beziehungsweise der Cantilever von der Basis in den verformbaren Teil übergeht, wird Biegekante genannt. Die Biegekante ist üblicherweise eine Kante des Substrats, wenn der Cantilever über die Basis hinausragt.

Wird der Cantilever verbogen, so ergeben sich Materialspannungen und Kräfte in oder auf dem Material des Cantilevers, welche gemessen werden können. Sofern eine solche Materialspannung und/oder Kraft gemessen werden kann, lässt sich darüber auf eine Verbiegung des Cantilevers schließen.

Die Transducer haben den Zweck, die Verformung der Cantilever zu bestimmen oder zu messen. Die aktiven Transducer sind auf den verformbaren Teilen der Cantilever angeordnet, wohingegen die passiven Transducer auf den Basen, beispielsweise den Basen, der Cantilever angeordnet sind. Insbesondere können über die Transducer elektrische Eigenschaften eines Schaltkreises beeinflusst werden.

Beispielsweise kann eine Verformung des Cantilevers dazu führen, dass der Widerstand eines Transducers, beispielsweise eines aktiven Transducers, ansteigt, während keine Verformung des Cantilevers auch keine Veränderung des Widerstands des Transducers hervorruft. Dies kann beispielsweise über eine Ausbildung der Transducer nach dem Prinzip eines Dehnungsmessstreifens erfolgen, wodurch sich eine Verformung des jeweiligen Cantilevers in einer Längenänderung des darauf aufgebrachten Dehnungsmessstreifens des Transducers äußert und damit eine Verformung des Cantilevers direkt durch eine Veränderung des Widerstands des Dehnungsmessstreifens detektiert werden kann.

Somit wird die chemische und/oder biochemische Information des Analyten über einer Verformung des Cantilevers, eine anschließende Registrierung über einen Transducer, und schließlich über eine Änderung einer elektrischen Eigenschaft eines Schaltkreises detektierbar.

Die Verformung der Cantilever kann durch eine geeignete Beschichtung stoffspezifisch herbeigeführt werden. Aus diesem Grund weist der Referenzcantilever eine Referenzschicht zur selektiven Nichtaufnahme des Analyten auf, während der Testcantilever eine Rezeptorschicht zur Aufnahme des Analyten aufweist.

Eine Rezeptorschicht ist hierbei ein Stoff, der mit dem Analyten in Wechselwirkung treten kann. Dies bedeutet wiederum, dass die Rezeptorschicht spezifisch für jeden Analyten gewählt wird. Analog ist eine Referenzschicht ein Stoff, der mit dem Analyten nicht in Wechselwirkung treten kann. Auch die Referenzschicht wird daher spezifisch für den Analyten gewählt.

Wechselwirkung bedeutet in diesem Fall, dass der Analyt in chemischer und/oder biochemischer und/oder physikalischer Wechselwirkung mit der Rezeptorschicht steht. Insbesondere kann die Wechselwirkung in einer Bindung des Analysten an die Rezeptorschicht bestehen. Eine Wechselwirkung kann außerdem in der Absorption oder Adsorption oder einer unspezifischen Adhäsion des Analyten an die Rezeptorschicht bestehen.

Die Rezeptor- und Referenzschichten sind bevorzugt chemisch identisch bezüglich möglicher Störeinflüsse und unterscheiden sich bevorzugt nur durch die Wechselwirkung mit dem Analyten. Ein Stoff, der nicht der Analyt ist, wechselwirkt dementsprechend genauso stark oder genauso schwach mit der Rezeptorschicht wie mit der Referenzschicht.

Die selektive Aufnahme des Analyten am Testcantilever bewirkt, dass eine Kraft durch den Analyten auf den Testcantilever wirkt, so dass der Testcantilever sensitiv auf den Analyten reagiert. Dementsprechend tragen die anderen Stoffe der Probe, die nicht der Analyt sind, lediglich zu einem Grundrauschen in Form einer Grundverbiegung am Testcantilever bei. Die Kraft auf den Testcantilever steigt beispielsweise umso schneller größer, je größer die Konzentration des Analyten in der Probe ist oder je schneller die Oberfläche des Cantilevers mit dem Analyten belegt ist. Eine für die jeweilige Ausbildung mögliche Maximalkraft wird bei einer vollständigen Belegung des Cantilevers erreicht.

Die selektive Nichtaufnahme des Analyten am Referenzcantilever bewirkt hingegen, dass keine Kraft durch den Analyten auf den Referenzcantilever wirkt, so dass nur die Stoffe, die nicht der Analyt sind, zu einem Grundrauschen in Form einer Grundverbiegung des Referenzcantilevers beitragen.

Diese wirkende Kraft kann bei dem verformbaren Teil des Testcantilevers eine Verformung bewirken, während der verformbare Teil des Referenzcantilever nicht verbogen wird. Grundlage für die Auslenkung des Cantilevers ist die Änderung der Oberflächenspannung durch die Wechselwirkung mit dem Analyten. Die Änderung der Oberflächenspannung führt zu einer Dehnung oder Stauchung der oberen (oder unteren) Oberfläche des Cantilevers. Die unterschiedliche Dehnung oder Stauchung an Ober- und Unterseite bewirkt in dem Material eine interne Kraft oder Materialspannung, die zur Verformung führt.

Referenzcantilever nach dem Stand der Technik weisen lediglich keine Rezeptorschicht auf, die sensitiv auf den Analyten reagiert. Dadurch können zwar Effekte wie Turbulenz in der Probe und die thermische Drift des Sensorsystems bestimmt werden. Jedoch kann bei einem solchen Referenzcantilever der Analyt beispielsweise durch eine unspezifische Bindung an die Referenzschicht des Referenzcantilevers binden. Dadurch trägt aber der Analyt selbst zum Grundrauschen bei. Daher sind bei einem Sensor nach dem Stand der Technik Referenzmessungen in einer Referenzprobe, also einer Probe ohne Analyten, notwendig. Nur dadurch lässt sich der Effekt der unspezifischen Bindung der Stoffe, die nicht der Analyt sind, feststellen.

Bei dem erfindungsgemäßen Sensor wird durch die selektive Nichtaufnahme des Analyten durch den Referenzcantilever das Messverfahren drastisch vereinfacht, da der Referenzcantilever nicht sensitiv für den Analyten ist und daher der Analyt auch nicht zum Grundrauschen beiträgt. Nur die Stoffe, die nicht der Analyt sind, tragen hier zum Grundrauschen des Referenzcantilevers bei. Gewissermaßen kann durch die selektive Nichtaufnahme des Analyten am Referenzcantilever bewirkt werden, dass der Referenzcantilever denselben Turbulenzen, derselben thermischen Drift und demselben Einfluss aller Stoffe, die nicht der Analyt sind, ausgesetzt ist, wie in einer Referenzprobe. Jedoch mit dem Unterschied, dass das Referenzsignal direkt in der Probenflüssigkeit bestimmt wird.

Insbesondere bewirkt ein Referenzcantilever mit Referenzschicht und ein Testcantilever mit Rezeptorschicht eine deutlich spezifischere Analyse des Analyten als lediglich ein Referenzcantilever ohne Rezeptorschicht, da sowohl die Referenzschicht als auch die Rezeptorschicht eine spezifische Wechselwirkung beziehungsweise Nicht-Wechselwirkung mit dem Analyten aufweisen.

Der Aufbau des Sensors mit Referenzcantilever und Testcantilever hat den Vorteil, dass in der Probe gleichzeitig zwei Messungen vorgenommen werden können, wobei die Messung des Referenzcantilevers die Messung des Testcantilevers kalibrieren kann. Dadurch lassen sich Umgebungseinflüsse, etwa chemische, thermische, mechanische, elektrische und fluidische Störeinflüsse, auf die jeweilige Messung reduzieren, so dass ein Vorkommen des Analyten aus dem Vergleich der Messung am Testcantilever und am Referenzcantilever geschlossen werden kann.

Diese Kräfte oder Materialspannungen, beispielsweise Dehnungen oder Stauchungen, die auf die Cantilever wirken, können schließlich von den Transducern detektiert werden, wobei durch unterschiedlich starke Dehnungen oder Stauchungen unterschiedlich starke Spannungen von den Transducer detektiert werden.

Bei dem hier vorgeschlagenen Sensor wird durch die selektive Nichtaufnahme des Analyten durch den Referenzcantilever das Messverfahren drastisch vereinfacht, da der Referenzcantilever nicht sensitiv für den Analyten ist, und daher der Analyt auch nicht zum Grundrauschen beiträgt. Nur die Stoffe, die nicht der Analyt sind, tragen hier zum Grundrauschen des Referenzcantilevers bei. Gewissermaßen kann durch die selektive Nichtaufnahme des Analyten am Referenzcantilever bewirkt werden, dass der Referenzcantilever denselben Turbulenzen, derselben thermischen Drift und demselben Einfluss aller Stoffe, die nicht der Analyt sind, ausgesetzt ist, wie der Testcantilever und auch wie in einer Referenzprobe. Jedoch mit dem Unterschied, dass das Referenzsignal direkt in der Probenflüssigkeit bestimmt wird.

Mit anderen Worten ist eine Kalibrierung des Referenzcantilevers in einer definierten Referenzprobe nicht mehr notwendig, sondern die Messung mit Testcantilever und Referenzcantilever kann direkt in der zu analysierenden Probe durchgeführt werden.

Insbesondere bewirkt ein Referenzcantilever mit Referenzschicht und ein Testcantilever mit Rezeptorschicht eine deutlich spezifischere Analyse des Analyten als lediglich ein Referenzcantilever ohne Referenzschicht, da sowohl die Referenzschicht als auch die Rezeptorschicht eine spezifische Wechselwirkung beziehungsweise Nicht-Wechselwirkung mit dem Analyten aufweisen.

Indem die aktiven Transducer auf den verformbaren Teilen der Cantilever angeordnet sind, kann über die aktiven Transducer ein Maß gefunden werden, welches der Stärke der Wechselwirkung des Analyten mit dem verformbaren Teil entspricht. Die passiven Transducer hingegen sind auf den Basen der Cantilever angeordnet, sodass sich die Wechselwirkung auf diejenigen Wechselwirkungen reduziert, die nicht primär eine Verformung des verformbaren Teils hervorrufen.

Der Aufbau des Sensors mit Referenzcantilever und Testcantilever hat den Vorteil, dass in der Probe gleichzeitig zwei Messungen vorgenommen werden können, wobei die Messung des Referenzcantilevers die Messung des Testcantilevers kalibrieren kann. Dadurch lassen sich Umgebungseinflüsse, etwa chemische, thermische, mechanische, elektrische und fluidische Störeinflüsse, auf die jeweilige Messung reduzieren, so dass auf ein Vorkommen des Analyten aus dem Vergleich der Messung am Testcantilever und am Referenzcantilever geschlossen werden kann.

Insbesondere ist es auch möglich, die Geometrie des Referenzcantilevers auf einen bestimmten Störeinfluss hin zu optimieren, um diesen Störeinfluss dann bei der Auswertung gezielt zu berücksichtigen. Es ist zudem auch möglich, unterschiedliche Störeinflüsse mit unterschiedlichen Referenzcantilevern zu bestimmen.

Mit anderen Worten wird auch ein Aufbau des Sensors mit einem Testcantilever und mindestens zwei Referenzcantilevern vorgeschlagen, wobei die unterschiedlichen Referenzcantilever dann bevorzugt für unterschiedliche Störeinflüsse optimiert sind.

Die selektive Aufnahme des Analyten durch die Rezeptorschicht und die selektive Nichtaufnahme des Analyten, bevorzugt des Virus durch die Referenzschicht kann eine relative Auslenkung des Testcantilevers zum Referenzcantilever verursachen, wobei durch Vergleich der durch die Transducer detektierten Kräfte auf das Vorkommen des Analyten, bevorzugt des Virus geschlossen wird, bevorzugt auf die Größe des Vorkommens geschlossen wird.

Gemäß dem vorgeschlagenen Sensor umfasst der Sensor eine Test-Funktionsschicht und/oder eine Referenz-Funktionsschicht, welche zwischen der Test-Wandlerschicht und der Rezeptorschicht, und/oder zwischen der Referenz-Wandlerschicht und der Referenzschicht angeordnet ist.

Die Funktionsschicht kann eine Vielzahl unterschiedlicher Funktionen erfüllen, beispielsweise eine Verbesserung der Sensitivität des Sensors durch Verschiebung der neutralen Achse und damit der Verbesserung der Dehnung am Ort der jeweiligen Transducer oder eine Verbesserung der Oberflächengüte zum Aufbringen der Rezeptorschicht oder der Referenzschicht oder zur Anpassung der Oberflächenenergie, so dass unterschiedliche Rezeptor- und Referenzschichten aufgebracht werden können, um entsprechend unterschiedliche Analyten zu detektieren. Diese exemplarisch genannten Funktionen werden im Weiteren detailliert beschrieben.

Die Funktionsschicht kann auch zur Abdeckung auf die Test-Wandlerschicht und/oder die Referenz-Wandlerschicht deponiert werden sodass diese zwischen der Test-Wandlerschicht bzw. der Referenz-Wandlerschicht und einer Rezeptorschicht bzw. einer Referenzschicht liegt.

Die Deposition und Strukturierung der Funktionsschichten kann dabei mit verschiedensten Methoden durchgeführt werden, wie beispielsweise durch Elektronenstrahlverdampfung, Sputter-Deposition, Elektronenstrahllithographie, Plasma-unterstützte chemische Gasphasenabscheidung (PECVD), chemische Gasphasenabscheidung (CVD), Atomlagenabscheidung (ALD), durch Aufschleudern mittels einer Lackschleuder, Inkjet-Printing, galvanische Methoden und/oder weiterer Methoden.

Typische Materialien für Funktionsschichten können binäre Siliziumverbindungen (SiOx, SiN, ) und/oder Metalloxide (AlOx, ) umfassen.

Insbesondere können als Materialien für die Funktionsschichten anorganische Materialien wie beispielsweise mindestens eines der Materialien Diamant, 3C-SiC, 6H-SiC, Si3N4, SiO2, SCSi (100), SCSi (110), Poly-Si, Wolfram, Aluminium, Nickel, Kupfer, Titan, Gold und/oder Ni-Fe verwendet werden.

Insbesondere können als Materialien für die Funktionsschichten auch organische Materialien wie SU8, Polyimide, Polyamide, PVDF, PMMA und/oder SAMs (self-assembled monolayers) verwendet werden.

Die Funktionsschichten können auch durch Elektronenstrahl-Bedampfen mit den folgenden Materialien hergestellt werden: Al, AlSiCu, Au, Cr, Ge, In, Mg, Mo, Ni, Pd, Pt, Ti, W, Si, SiO.

Die Funktionsschichten können auch durch DC-Sputtern mit den folgenden Materialien hergestellt werden: Al, AlCu(96/4), Co, Cr, CrNi(50/50), Cu, Mo, Ni, NiV7, Si, Sn, Ti, W, WTi(90/10), MoSi/SiC.

Als Funktionsschichten können auch dielektrische Schichten mit den folgenden Materialien aufgebracht werden: Si3N4, SiO2, DLC, AlN, a-Si.

Bevorzugt ist die komplette Test-Wandlerschicht und/oder Referenz-Wandlerschicht vollflächig mit der Test-Funktionsschicht und/oder der Referenz-Funktionsschicht abgedeckt und bedeckt damit alle Transducer. Alternativ kann die Abdeckung durch die Funktionsschichten auch nur einen Teil der Wandlerschichten umfassen, wobei bevorzugt mindestens einer der jeweiligen Transducer des jeweiligen Cantilevers abgedeckt ist.

Wird die Test-Wandlerschicht und/oder die Referenz-Wandlerschicht mit einer jeweiligen Funktionsschicht abgedeckt, kann eine zur Aufnahme eines Analyten konzipierte Rezeptorschicht bzw. Referenzschicht nicht unmittelbar auf die Test-Wandlerschicht bzw. Referenz-Wandlerschicht aufgetragen werden, da ansonsten kein Kontakt der Rezeptorschicht bzw. Referenzschicht mit der Probe und damit mit dem Analyten zustande kommen würde. Dementsprechend wird für die Detektionsfähigkeit des Sensors eines Analyten, welcher Funktionsschichten gemäß der vorliegenden Offenbarung umfasst, eine entsprechende Rezeptorschicht bzw. Referenzschicht auf die außenliegende Oberfläche der Test-Funktionsschicht bzw. Referenz-Funktionsschicht aufgebracht. Dies hat insbesondere den Vorteil, dass die Funktionsschichten bezüglich ihrer Oberfläche auf die darauf deponierten Rezeptor- bzw. Referenzschichten optimiert werden können. Details hierzu werden später diskutiert.

Die Abdeckung der Wandlerschichten mittels der Funktionsschichten verhindert einen unmittelbaren Kontakt zu jeglichen umliegenden Stoffen, Flüssigkeiten oder Gasen, die im Falle ohne abdeckende Funktionsschichten einen direkten Kontakt zu den Wandlerschichten hätten.

Zu den äußeren Einflüssen zählen beispielsweise die die Wandlerschichten umgebende (Luft-) Feuchte, Temperaturänderungen der Umgebung der Wandlerschichten oder der pH-Wert oder die Salze/Ionenkonzentration von die Wandlerschichten umgebenden Medien. Im Allgemeinen beeinflusst jeglicher polare Stoff in einer Lösung die Dielektrizität der Lösung, und wird daher zu einem äußeren Einfluss gezählt. Weiterhin ist auch der Einfluss von Luftgasen in der die Wandlerschichten umgebenden Luft oder die in der Probe gelösten Analyten zu nennen. Insbesondere ist hierbei die endliche Leitfähigkeit von Lösungen zu nennen, die die Wandlerschichten umgeben und in denen die Analyten gelöst sind. Im Allgemeinen führt der Kontakt der Wandlerschichten, insbesondere der darin eingebetteten Transducer, zu leitfähigen Medien zu einem größeren Rauschen des Sensors und sollte daher bevorzugt vermieden werden.

Die zuvor genannten Einflüsse bedingen bei fehlenden Funktionsschichten insbesondere einen verstärkten Drift des Sensorsignals. Drift des Sensorsignals bedeutet dabei, dass die zu detektierende elektrische Größe der Transducer sich über die Zeit hinweg ändert, weil sich die äußeren Einflüsse auf die Transducer ändern, obwohl sich das Vorliegen oder die Konzentration des Analyten nicht geändert hat. Mit anderen Worten kann durch eine Veränderung der äußeren Einflüsse, die nicht der Analyt sind, eine Veränderung des Sensorsignals einhergehen. Dies kann dazu führen, dass fälschlich auf eine Veränderung im Vorliegen oder der Konzentration des Analyten oder auf ein Nichtvorliegen des Analyten geschlossen wird. Durch das Vorsehen der hier vorgeschlagenen Funktionsschicht oder der Funktionsschichten kann eine solche Drift des Sensorsignals aufgrund von äußeren Einflüssen reduziert oder vermieden werden, so dass die Genauigkeit und Zuverlässigkeit der Detektion des Analyten verbessert wird.

Die externen Einflüsse können auch zu stärkerem Rauschen des Sensors führen, wodurch das Signal-zu-Rausch Verhältnis verschlechtert wird oder der Sensor gar seine Sensitivität verliert. Durch die vorgeschlagene Funktionsschicht oder die Funktionsschichten kann das Signal-zu-Rausch Verhältnis konstanter oder konstant gehalten werden.

Zudem können die genannten äußeren Einflüsse Alterungseffekte der Transducer bedingen, welche die entsprechende elektrische Messgröße, die als Parameter zur Detektion des Analyten in der Probe benutzt wird, dauerhaft derartig verändert, dass ein gefahrloser beziehungsweise zuverlässiger Betrieb des Sensors nicht mehr möglich ist. Durch die vorgeschlagene Funktionsschicht oder die Funktionsschichten können auch Alterungseffekte der Transducer reduziert oder vermieden werden, so dass der Sensor eine zuverlässigere Detektion des Analyten ermöglicht.

Entsprechend weisen die abgedeckten Transducer durch die schützenden Funktionsschichten, insbesondere durch den unterdrückten Sauerstoffkontakt, Luftfeuchte, etc., keine bzw. keine wesentlichen Alterungseffekte, wie beispielsweise Drift des Sensorsignals und/oder Verschlechterung des Signal-zu-Rausch Verhältnisses, auf.

Weiterhin können die Funktionsschichten die Wandlerschichten vor äußeren Einflüssen schützen, welche beispielsweise durch die Wandlerschichten umgebende reaktive Medien induziert sind, insbesondere durch Säuren oder reaktive Gase. Dies verhindert insbesondere so genanntes chemoristisches Verhalten der Transducer. Dabei bedeutet chemoristisches Verhalten, dass ein Material als Antwort auf eine sich in der Nähe beziehungsweise mit dem Material in Kontakt befindlichen chemischen Umgebung seinen elektrischen Widerstand ändert.

Nicht ausgeschlossen ist aber die Verwendung von porösen Funktionsschichten, die dadurch gekennzeichnet sind, dass eine Teildurchlässigkeit der Funktionsschichten für bestimmte Stoffe gegeben ist. Entsprechend kann chemoristisches Verhalten gezielt ausgenutzt werden, indem von einer porösen Funktionsschicht abgedeckte Transducer eine gezielte Sensitivität bezüglich eines oder mehrerer Analyten erhalten, welche durch die poröse Funktionsschicht hindurch zum Transducer gelangen können. Dabei können beispielsweise auch verschiedene Funktionsschichten mit unterschiedlichen Eigenschaften bezüglich ihrer Teildurchlässigkeit verwendet werden.

Bevorzugt sind die Funktionsschichten aus einem elektrisch isolierenden Material mit einem hohen Isolationswiderstand hergestellt. Dies ist besonders bevorzugt für die Funktionsschicht oder die Funktionsschichten der Fall, die in direktem Kontakt mit den Transducern und/oder den Elektroden stehen. Weiterhin können die Test-Funktionsschicht und die Referenz-Funktionsschicht aus demselben Material hergestellt sein.

Zum Betrieb der Transducer, insbesondere zum Auslesen der zu detektierenden elektrischen Messgröße, sind typischerweise elektrische Spannungen nötig, welche über die Elektroden der Transducer abfallen. Im Falle einer fehlenden isolierenden Test-Funktionsschicht bzw. Referenz-Funktionsschicht, welche die Test-Wandlerschicht bzw. die Referenz-Wandlerschicht abdeckt, haben die mit den Spannungen verbundenen elektrischen Potentiale einen direkten Einfluss auf die Test-Wandlerschicht bzw. die Referenz-Wandlerschicht, insbesondere an den Elektroden der Transducer.

Da die Wandlerschichten ohne isolierende Funktionsschichten für die Detektionsfähigkeit des Sensors eines Analyten auch eine Rezeptorschicht bzw. Referenzschicht zur selektiven Aufnahme bzw. Nicht-Aufnahme eines zu detektierenden Analyten umfassen müsste, würden die Bindemoleküle der Rezeptorschicht (beispielsweise Antikörper) bzw. Referenzschicht sowie die auf den Wandlerschichten gebundenen Analyten ebenfalls von den in der Wandlerschichten bestehenden elektrischen Potentialen, beispielsweise in ihren Bindeeigenschaften (Spezifizität, Bindungsraten, Bindungsvermögen etc) beeinflusst werden.

Wird beispielsweise ein Testcantilever und/oder Referenzcantilever mit Test-Wandlerschicht und/oder Referenz-Wandlerschicht sowie einer Rezeptorschicht und/oder Referenzschicht, aber ohne schützende Test-Funktionsschicht und/oder Referenz-Funktionsschicht in Kontakt mit einer Probe gebracht, kann die über die Elektroden der aktiven und/oder passiven Transducer abfallende Spannung neben dem elektrischen Strom, der über die Transducer fließt, einen geringen, aber endlichen Leckstrom zur Folge haben, welcher beispielsweise über den hohen, aber endlichen Widerstand des Probenmaterials, beispielsweise einer flüssigen Lösung, fließt. Die in der Probe befindlichen Chemikalien, sowie die Bindemoleküle der Rezeptorschicht und/oder Referenzschicht (welche selbst Chemikalien sind) sind dann einer elektrischen Spannung bzw. einem elektrischen Leckstrom ausgesetzt, welcher zu elektrochemischen Reaktionen der beteiligten Chemikalien führen kann. Entsprechend können elektrochemisch induzierte Änderungen besagter Chemikalien einen Einfluss auf die elektrischen Signale der Transducer haben.

Isolierende Funktionsschichten, welche die Wandlerschichten abdecken, haben nun den entscheidenden Vorteil, dass elektrochemische Reaktionen, welche im Falle ohne isolierende Funktionsschicht insbesondere an den Elektroden der Transducer durch den direkten Kontakt der Transducer zur Probe entstehen würden, unterdrückt werden. Mit anderen Worten ist durch die Funktionsschichten die Oberfläche der Funktionsschichten, welche durch entsprechend konzipierte Folgeschichten (wie beispielsweise einer Rezeptorschicht oder einer Referenzschicht) zur Aufnahme oder Nicht-Aufnahme eines spezifischen Analyten eingerichtet ist, im Wesentlichen frei von elektrischen Potentialen und damit von elektrochemischen Reaktionen.

Die Abwesenheit von elektrochemischen Reaktionen in den Wandlerschichten hat zur Folge, dass elektrische Signale der Transducer nicht mehr durch derartige Reaktionen beeinflusst werden können und diejenigen Änderungen der elektrischen Signale, welche durch den zu untersuchenden Analyten induziert sind, nicht mehr durch elektrochemisch induzierte Signale überlagert sind. Dadurch verbessert sich die Sensitivität der Transducer und damit des Sensors. Insbesondere verbessert sich das Rauschverhalten der Transducer, wodurch das Signal-zu-Rausch Verhältnis des Sensors verbessert wird.

Durch die zwischen der Test-Wandlerschicht und/oder der Referenz-Wandlerschicht und der Rezeptorschicht und/oder der Referenz-Schicht angeordnete Test-Funktionsschicht und/oder Referenz-Funktionsschicht ist die Rezeptorschicht und/oder die Referenzschicht und dessen Bindemoleküle, insbesondere Proteine wie beispielsweise Antikörper, von den über den Transducern beziehungsweise entlang der Wandlerschichten abfallenden elektrischen Spannungen isoliert. Dementsprechend ist ein durch elektrische Spannungen induzierter Einfluss auf die Rezeptorschicht und/oder Referenzschicht und dessen Bindemoleküle aufgrund elektrochemischer Reaktionen unterdrückt.

Bei der Beschichtung eines Cantilevers mit einer Rezeptorschicht oder Referenzschicht, bei welchem die Rezeptorschicht oder Referenzschicht in direktem Kontakt mit der jeweiligen Wandlerschicht und dadurch mit dem aktiven und/oder passiven Transducer steht, kann es zu Störeinflüssen der Transducer kommen. Unter Verwendung der Funktionsschichten, die zwischen den Wandlerschichten und der Rezeptorschicht bzw. Referenzschicht angeordnet ist, werden derartige Störeinflüsse durch die Beschichtung unterdrückt.

Der durch die Funktionsschichten überdeckte Bereich der Wandlerschicht kann zudem als Schutz vor elektrostatischen Entladungseffekten in den Wandlerschichten dienen. Eine der Umgebung exponierte Wandlerschicht, welche keine schützenden Funktionsschichten aufweist, kann insbesondere bei einer elektrisch leitfähigen Schicht zur elektrischen Kontaktierung der Transducer anfällig für elektrische Entladungen sein, die sich durch etwaige elektrische Potentiale in der unmittelbaren Umgebung der Wandlerschichten ergeben können. Die die Wandlerschicht überdeckenden isolierenden Funktionsschichten können besagte Entladungen über die leitfähige Schicht der Wandlerschichten beziehungsweise über die Elektroden der Transducer verhindern. Ein solcher Schutz ist vor allem bei Ausführungsformen mit hohen Isolationswiderständen der Funktionsschichten stark ausgeprägt, und kann Entladungen auch bei hohen Potentialen verhindern.

In einer besonders bevorzugten Ausführungsform sind die Wandlerschichten als elektrisch leitfähige Schichten ausgeführt, die zu Elektroden strukturiert sind, sodass die aktiven und passiven Transducer extern elektrisch kontaktiert werden können. Ein besonderer Vorteil einer die Wandlerschichten überdeckenden Funktionsschichten besteht darin, dass auch nicht-inerte Materialien als leitfähige Schichten geeignet sind, da durch die Funktionsschichten keine Exposition zur Umgebung besteht und insbesondere die Oxidation von reaktiven Materialien unterdrückt wird.

Weiterhin können die Wandlerschichten neben leitfähigen Schichten wie oben beschrieben auch Aktivierungsschichten umfassen, die dazu eingerichtet sind, eine entlang der Wandlerschichten vergrößerte Oberflächenspannung herbeizuführen. Durch die überdeckenden Funktionsschichten wird es ermöglicht, auch nicht-inerte Materialien als Aktivierungsschicht zu verwenden.

In einer besonders bevorzugten Ausführungsform können die als Aktivierungsschichten ausgeführten Wandlerschichten gleichzeitig als elektrisch leitfähige Schichten zur elektrischen Kontaktierung der Transducer fungieren. Entsprechend kann die Aktivierungsschicht aus einem elektrisch leitenden Material hergestellt sein, insbesondere aus Gold, wobei typischerweise aufgrund der nur schlecht benetzenden Eigenschaft von Gold auf der Oberfläche des Cantilevers ein Haftvermittler, wie beispielsweise Titan, zwischen der Goldschicht und dem Cantilevermaterial benutzt wird. Wie oben beschrieben sind jedoch durch die schützenden Funktionsschichten auch nicht-inerte Materialien geeignet. Entsprechend ist die Auswahl an Materialien für Aktivierungsschichten signifikant erhöht und bei geeigneter Materialwahl kann auch auf einen Haftvermittler verzichtet werden.

Eine weitere Funktion der Funktionsschichten liegt darin, die Variabilität des Sensors bezüglich geeigneter Rezeptorschichten bzw. Referenzschichten zur spezifischen Detektion bzw. Nicht-Detektion eines Analyten in einer Probe zu verbessern sowie die Sensitivität des Sensors zu erhöhen.

Die Aufgabe wird ebenfalls durch eine zwischen den Wandlerschichten und der Rezeptorschicht bzw. Referenzschicht angeordneten Funktionsschichten gelöst.

Im Falle fehlender Funktionsschichten müssen die Wandlerschichten der Cantilever bestimmte Eigenschaften aufweisen, damit eine nachfolgende Rezeptorschicht bzw. Referenzschicht die darunterliegenden Wandlerschichten derartig benetzt, dass eine geeignete Haftung zwischen den Schichten sichergestellt ist.

Eine Haftung/Benetzung einer Schicht erfordert, dass die Oberflächenenergie der zu benetzenden Schicht deutlich größer ist als die Oberflächenenergie der benetzenden Schicht. Entsprechend müssen im Fall ohne Funktionsschichten die Wandlerschichten neben der Aktivierungseigenschaft und/oder der elektrisch leitfähigen Eigenschaft und/oder der inerten Eigenschaft auch die Oberflächenenergie dergestalt sein, dass eine nachfolgende Rezeptorschicht bzw. Referenzschicht an der jeweiligen Wandlerschicht haftet. Dies schränkt die Wahl geeigneter Materialen für die Wandlerschichten und der Rezeptorschicht bzw. Referenzschicht signifikant ein.

Werden entsprechend Funktionsschichten zwischen den Wandlerschichten und der Rezeptorschicht bzw. Referenzschicht angeordnet, muss lediglich die Oberflächenenergie der Funktionsschichten auf die Oberflächenenergie der Rezeptorschicht bzw. Referenzschicht angepasst werden, um eine geeignete Benetzung/Haftung zwischen den Schichten zu gewährleisten. Dadurch wird die Auswahlmöglichkeit der Wandlerschichten, wie weiter oben bereits ausgeführt, aber auch die Wahlmöglichkeiten der Rezeptorschichten bzw. Referenzschichten signifikant erhöht. Insbesondere können die Funktionsschichten auf beliebige Rezeptorschichten bzw. Referenzschichten angepasst werden.

Gemäß einer typischen Ausführungsform kann eine Passivierungsschicht auf der unteren Oberfläche der Cantilever angebracht sein. Die Passivierungsschicht ist dazu eingerichtet, eine unspezifische Adhäsion eines Analyten auf der Unterseite eines Cantilevers zu minimieren.

Es ist in einer bevorzugten Ausführungsform aber auch möglich, dass sich auf den Funktionsschichten als auch auf der Unterseite der jeweiligen Cantilever eine Rezeptorschicht zur selektiven Aufnahme eines Analyten befindet, wobei die durch Bindung eines Analyten an die Rezeptorschichten veränderte Oberflächenspannung der oberen Schicht zur Veränderung der Oberflächenspannung der unteren Schicht entgegengesetzt ist.

Gemäß dieser Ausführungsform verstärkt sich die Verformung durch die an die Bindemoleküle der Rezeptorschichten bindenden Analyten der oberen und unteren Oberfläche des Cantilevers, sodass die von dem aktiven Testtransducer erzeugten elektrischen Signale ebenfalls verstärkt sind, wodurch die Sensitivität des Sensors erhöht ist.

Weiterhin kann der mechanische Spannungszustand des Cantilevers durch den vorgeschlagenen Aufbau gezielt eingestellt werden.

In einem besonders bevorzugten Fall ist das Spannungsfeld eines Cantilevers bei einer Verformung desselben derart konfiguriert, dass die Dehnung/Stauchung am Ort des aktiven Testtransducers und/oder des aktiven Referenztransducers maximal ist, sodass auch die elektrischen Signale des aktiven Testtransducers und/oder aktiven Referenztransducers maximiert werden.

Dabei ist sowohl eine Längs- als auch eine Querdehnung der Cantilever, sowie beliebige Kombinationen der beiden miteingeschlossen. Dementsprechend sind die Transducer bevorzugt dazu eingerichtet, auf beide Dehnungsrichtungen sensitiv zu sein. Dabei umfassen die Begriffe Dehnung oder Verformung entsprechend Längs- als auch Querdehnungen sowie Mischdehnungen.

Die neutrale Ebene entspricht derjenigen gedachten Flächenkontur innerhalb eines Materials, beispielsweise eines Cantilevers, entlang welcher durch eine Oberflächenspannung auf dem Material keine Dehnung/Stauchung auftritt. Die neutrale Ebene wird durch zwei zueinander senkrechte neutrale Achsen aufgespannt. Dabei entsprechen die neutralen Achsen derjenigen gedachten Linien entlang der Längs- bzw. Querrichtung, welche durch eine Längs- bzw. Querspannung entlang der Oberfläche des Cantilevers keine Dehnung/Stauchung erfahren. Entsprechend unterliegen Stellen, welche besonders weit entfernt von der neutralen Ebene bzw. den Achsen positioniert sind, einer besonders großen Dehnung/Stauchung.

Mit Hilfe der Funktionsschichten, welche auf den Wandlerschichten angeordnet sind und daher die Transducer beziehungsweise vereinzelte Transducer abdecken, kann die Dehnungsverteilung der Cantilever zusätzlich eingestellt werden.

Betrachtet man exemplarisch die Dehnung entlang einer ausgewählten Richtung, beispielsweise entlang der Längsrichtung des Cantilevers, kann die Lage der longitudinalen neutralen Achse entlang besagter Richtung quantitativ angegeben werden. Entsprechend kann die geometrische Lage $\overline{y}$ der neutralen Achse für eine Struktur bestehend aus zwei Schichten mit Dicken $h_1$ und $h_2$ ausgedrückt werden über

$$\overline{y} = \frac{h_1 + h_2}{2}\left(\frac{\lambda}{1 + \lambda} + \frac{E_2}{E_2 + \lambda\,E_1}\right),$$

wobei $\lambda = h_1/h_2$ das Dickenverhältnis der Schichten ist und $E_1$ bzw. $E_2$ die Elastizitätsmodule der Schichten darstellt. Für isotrope Materialien, deren Elastizitätsmodule ebenfalls isotrop sind, ist die Lage der transversalen neutralen Achse entsprechend äquivalent und es ergibt sich eine flache Kontur der Ebene (vorausgesetzt die Dicken der Schichten sind homogen). Anisotrope Materialien mit entsprechend anisotropen Elastizitätsmoduln führen aber zu einer nicht-trivialen Kontur der neutralen Ebene.

Entsprechend kann nach obiger Gleichung beispielsweise Elastizitätsmodul der Funktionsschichten so gewählt werden, dass die neutrale Ebene bzw. die neutralen Achsen der Cantilever nach unten, also in eine Richtung weg von den Wandlerschichten beziehungsweise der Transducer, verschoben wird, sodass die Dehnung am Ort der Transducer unter Verformung maximiert wird. Für diese Ausführungsform muss der Elastizitätsmodul der Funktionsschichten also ausreichend viel kleiner als der mittlere Elastizitätmodul der darunterliegenden Schichten gewählt werden. Eine durch unterschiedliche Elastizitätsmodule von verschiedenen Schichten induzierte Spannung in den Cantilevern wird auch als intrinsische Vorspannung oder Eigenspannung bezeichnet.

Vorzugsweise kann der Elastizitätsmodul der Funktionsschichten besonders klein gewählt werden, insbesondere kleiner als der Elastizitätsmodul der darunterliegenden Wandlerschichten und/oder des Grundkörpers der Cantilever. Im Allgemeinen kann der Elastizitätsmodul der Funktionsschichten aber nach Belieben gewählt werden, um einen bestimmte Spannnungsverteilung des Cantilevers unter Verformung hervorzurufen bzw. einzustellen.

Auch kann nach obiger Gleichung die Dicke der Funktionsschichten benutzt werden, um die Lage der neutralen Ebene bzw. Achsen der Cantilever zu beeinflussen. Dabei verschiebt sich die neutrale Ebene bei größer werdenden Dicken in Richtung der Funktionsschichten, also zu den Wandlerschichten hin. Dementsprechend sollten für den umgekehrten Effekt kleinere Dicken gewählt werden.

Entsprechend kann in einer weiteren Ausführungsform durch geeignete Strukturierung der Funktionsschichten eine durch Verformung des verformbaren Teils der Cantilever induzierte Spannungsverteilung in einem Cantilever entlang der Wandlerschichten, insbesondere am Ort der aktiven Transducer, maximiert werden. Mit Strukturierung der Funktionsschichten ist hierbei eine gezielte Veränderung des Höhenprofils der Funktionsschichten gemeint.

Beispielsweise können die Funktionsschichten am Ort der Transducer durch Strukturierung erreicht werden, wobei das Höhenprofil der Funktionsschichten derart verändert wird, dass der Spannungszustand am Ort der Transducer maximiert wird. Dies kann zum Beispiel dadurch erfolgen, dass die Dicke der Funktionsschichten, beispielsweise durch gezieltes lokales Ätzen der Funktionsschichten am Ort der Transducer, verringert wird, sodass sich die neutrale Ebene des jeweiligen Cantilevers an besagten Orten vom Transducer weg verschiebt, wodurch die Dehnung am Ort der Transducer unter Verformung erhöht wird.

Durch Aufbringen von Aktivierungsschichten können die jeweiligen Cantilever auch einen Spannungszustand aufweisen, welcher zu einer derart starken intrinsischen Vorspannung führt, dass der jeweilige Cantilever selbst im Gleichgewichtszustand, also ohne an die Rezeptorschicht bzw. Referenzschicht bindende Analyten, stark verbogen ist.

In diesem Fall ist es von Vorteil, wenn der Spannungszustand in einen neutralen, bevorzugt geraden Gleichgewichtszustand der Cantilever überführt werden kann. Letzteres kann über die zusätzlichen Funktionsschichten erreicht werden, indem die Materialeigenschaften der Funktionsschichten, insbesondere deren Dicke und/oder Elastizitätsmodul, derart eingestellt werden, dass ein verbogener Zustand der Cantilever in einen neutralen, bevorzugt geraden, Zustand überführt wird.

Weiterhin kann auch eine geeignete Strukturierung der Funktionsschichten verwendet werden, um einen im Gleichgewicht spannungsfreien Zustand der Cantilever zu erreichen, also einer etwaigen intrinsischen Vorspannung in den Cantilevern entgegenzuwirken.

Zur weiteren Optimierung, insbesondere Maximierung der Verspannung bzw. Längenänderung der Cantilever, insbesondere an der Position der Transducer, kann in einer weiteren Ausführungsform auch die Struktur der Transducer selbst beeinflusst werden, um den Spannungszustand der Transducer bei einer Verbiegung der Cantilever einzustellen.

Hierfür ist es sinnhaft, die strukturell bedingte Widerstandsänderung der Transducer zu quantifizieren. Der k-Wert, auch Gauge-faktor genannt, ist die Proportionalitätskonstante zwischen der Dehnung des Transducers und dessen Widerstandsänderung:

$$\frac{\Delta R}{R} = k \left( G_L \frac{\Delta L_l}{L_l} + G_T \frac{\Delta L_t}{L_t} \right),$$

wobei $\Delta R$ die Widerstandsänderung des Transducers ist, $R$ der Widerstand des Transducers bei unverbogenem Cantilever, $\Delta L_l$ und $\Delta L_t$ sind die longitudinale und transversale Längenänderung des Transducers, während $L_l$ und $L_t$ die longitudinale und transversale Länge des Transducers bei unverbogenem Cantilever ist. Die zueinander orthogonalen Längenänderungen sind dabei durch die Wichtungsfaktoren $G_L$ und $G_R$ gewichtet, wobei diese wiederrum durch die geometrische Konfiguration der Transducer bestimmt sind. Insbesondere können auch alle anderen zum k-Wert beziehungsweise zum Widerstand proportionalen Messgrößen, wie beispielsweise die Leitfähigkeit, gemessen werden.

Der k-Wert kann bei einem anisotropen Material auch richtungsgebunden sein.

Einerseits können die Transducer bezüglich ihrer Form in der Ebene der Wandlerschichten strukturiert werden. Durch eine Formänderung der Transducer in der Ebene kann eine Spannungsverteilung erzeugt werden, welche ein größeres elektrisches Signal bei einer Verformung der Cantilever erzeugt. Entsprechend kann die Strukturierung so gewählt werden, dass die Längenänderung an den Positionen der Transducer bei Verformung der Cantilever maximiert ist.

Im Speziellen können nach obenstehender Gleichung die geometrischen Wichtungsfaktoren $G_L$ und $G_T$ direkt durch eine Strukturierung der Transducer wie voranstehend beschrieben beeinflusst werden. Je nach gewünschter Sensitivität entlang Längs- und/oder Querrichtung, kann eine geeignete Strukturierung der Transducer vorgenommen werden, um die Wichtungsfaktoren zu erhöhen oder erniedrigen.

Andererseits können die Transducer auch bezüglich ihres Höhenprofils strukturiert sein Insbesondere ist in diesem Fall das Höhenprofil von einem Transducer derart gestaltet, dass es von einem flachen Profil abweicht. Durch eine Strukturierung des Höhenprofils des Sensors kann eine sehr gezielte Beeinflussung des Spannungszustandes des Transducers erreicht werden.

Wie zuvor beschrieben, induziert eine durch die Strukturierung erzeugte variierende Dicke der Transducer eine Verschiebung der neutralen Ebene bzw. Achsen, und dadurch eine Veränderung des Spannungszustands bzw. der Längenänderung am Ort der Transducer wenn eine extern induzierte Verbiegung der Cantilever eintritt. Bevorzugt wird das Höhenprofil derart gestaltet, dass die Längenänderung der Transducer bei einer Verbiegung der Cantilever maximiert ist.

Es ist ebenso möglich, die Transducer bezüglich ihrer Struktur in der Ebene der Wandlerschichten als auch bezüglich ihrem Höhenprofil gleichzeitig zu strukturieren, um eine gewünschte Spannungsverteilung in den Transducern zu erreichen.

Die Referenz- und Testcantilever können eine weitere Schicht aufweisen die eine selbstorganisierende Monoschicht umfasst.

Eine selbstorganisierende Monoschicht kann insbesondere Unebenheiten auf der darunterliegenden Oberfläche reduzieren und chemische Variationen ausgleichen, so dass eine gleichmäßige Beschichtung der Cantilever mit der Rezeptor- beziehungsweise Referenzschicht möglich ist. Durch die homogenen Oberflächeneigenschaften der Cantilever können sich so schließlich die Bindungseigenschaften der Rezeptorschicht und der Analyten verbessern.

Entsprechend kann die selbstorganisierende Monoschicht zwischen der Test-Funktionsschicht und/oder der Referenz-Funktionsschicht und der Rezeptorschicht und/oder der Referenzschicht angeordnet sein.

Die Transducer können dazu ausgebildet und eingerichtet sein, Verformungen der verformbaren Teile der Test- und des Referenzcantilever zu ermitteln, bevorzugt die jeweils auf die Basen und die verformbaren Teile der Test- und Referenzcantilever bei der Verformung ausgeübten Kräfte zu detektieren.

Wird ein Sensor, bestehend aus einem Referenzcantilever und einem Testcantilever mit der Referenzschicht beziehungsweise der Rezeptorschicht, einer Probe ausgesetzt die einen Analyten umfasst, so kann die Wechselwirkung in einer Bindung des Analysten an die Rezeptorschicht bestehen.

Durch die Bindung des Analyten an die Rezeptorschicht wird die Oberflächenspannung der mit der Rezeptorschicht belegten Seite des Testcantilevers verändert, was zu einer Kraft auf den Testcantilever führt, wohingegen keine Kraft durch den Analyten auf dem Referenzcantilever wirkt. Die Kraft auf den Testcantilever steigt beispielsweise umso schneller, je größer die Konzentration des Analyten in der Probe ist oder je schneller die Oberfläche des Cantilevers mit dem Analyten belegt ist. Um die Konzentration des Analyten zu messen, kann also beispielsweise die Steigung der über die Zeit kontinuierlichen Kraftänderung auf dem Testcantilever gemessen werden. Eine für die jeweilige Ausbildung mögliche Maximalkraft wird bei einer vollständigen Belegung des Cantilevers erreicht.

Diese Wechselwirkung kann bei dem verformbaren Teil des Testcantilevers eine Verformung bewirken, während der verformbare Teil des Referenzcantilever nicht verbogen wird.

Grundlage für die Auslenkung des Cantilevers ist die Änderung der Oberflächenspannung durch die Wechselwirkung mit dem Analyten. Die Änderung der Oberflächenspannung führt zu einer Dehnung oder Stauchung der oberen (oder unteren) Oberfläche des Cantilevers. Die unterschiedliche Dehnung oder Stauchung an Ober- und Unterseite bewirkt in dem Material eine interne Kraft oder Materialspannung, die zur Verformung führt.

Diese Kräfte oder Materialspannungen und insbesondere Dehnungen oder Stauchungen können schließlich von den Transducern detektiert werden, wobei durch unterschiedlich starke Dehnungen oder Stauchungen unterschiedlich starke Spannungen von den Transducern detektiert werden. Der jeweilige Transducer ändert entsprechend der Dehnungen oder Stauchungen seinen Widerstand, so dass auf diese Weise der Transducer zur Detektion der Dehnungen oder Stauchungen dient.

Die zu detektierende Kraft kann eine Biegekraft und/oder eine Dehnungskraft und/oder eine Stauchungskraft und/oder eine Scherkraft sein und/oder auf dem Elastizitätsmodul der Referenzcantilever und Testcantilever beruhen. Im Allgemeinen ist die zu detektierende Kraft also durch eine Oberflächenspannung auf der Oberfläche der Cantilever induziert.

Eine Biegekraft kann eine Veränderung der Geometrie des Cantilevers hervorrufen, insbesondere dem Cantilever eine Krümmung aufprägen, die sich vom unbeanspruchten Cantilever unterscheidet. Eine solche Krümmung kann zum Auftreten von Biegemomenten beziehungsweise Dehnungen und somit zu Biegespannungen führen, die mit einem entsprechenden Transducer bestimmt werden können.

Die Veränderung der Oberflächenspannung und die resultierende Dehnungskraft kann insbesondere eine Längenänderung des Cantilevers in diesem Bereich hervorrufen. Die Dehnung kann insbesondere an der oberen Oberfläche unterschiedlich zu unteren Oberfläche des Cantilever sein. Die Dehnung der Oberfläche kann insbesondere parallel zur Basis des Cantilevers erfolgen (eine sogenannte Querdehnung) oder senkrecht zur Basis des Cantilevers (eine sogenannte Längsdehnung) oder eine Kombination beider Richtungen. Die Größe der Dehnung hängt hierbei stark von der Geometrie der Cantilever sowie der weiteren auf den Oberflächen vorgesehenen Schichten, beispielsweise der Elektroden, ab, so dass eine optimale Detektion des Analyten durch eine Optimierung von Ausrichtung und Cantilevergeometrie erreicht werden kann. Insbesondere ist hierbei die Länge, Breite und das Dickenprofil aller beteiligten Schichten des Cantilevers von Bedeutung. Die jeweilige Längenänderung kann je nach Richtung des Kristallgitters des Cantilevers unterschiedlich sein.

Die relative Verformung und/oder die relative Veränderung der Oberflächenspannung verläuft bei unstrukturierten Transducern in einer Längsrichtung (also in Richtung der Längsdehnung) des Testcantilevers und/oder des Referenzcantilevers, wobei die Längsrichtung senkrecht zur Basis des Testcantilevers und/oder des Referenzcantilevers verläuft, wobei bevorzugt der aktive und der passive Testtransducer und/oder der aktive und der passive Referenztransducer in der Längsrichtung ausgerichtet sind und dadurch entlang besagter Richtung maximal sensitiv sind.

Besonders bevorzugt ist jedoch der Fall strukturierter Transducer wie weiter oben beschrieben, für welche die relative Verformung und/oder die relative Veränderung der Oberflächenspannung in Längs- und Querrichtung des Testcantilevers und/oder des Referenzcantilevers verläuft, wobei bevorzugt der aktive und der passive Testtransducer und/oder der aktive und der passive Referenztransducer durch eine geeignete geometrische Strukturierung auf beide Richtungen bezüglich ihrer Sensitivität optimiert sind.

Sofern die Dehnungskraft an der oberen Oberfläche und der unteren Oberfläche unterschiedlich ist, bezeichnet man die wirkende Kraft auch als Scherkraft.

Bei einem durchgebogenen Cantilever wirkt eine Biegekraft. Dadurch wird die obere Oberfläche des Cantilevers gedehnt und diese Dehnung ist insbesondere größer als an der unteren Oberfläche des Cantilevers (welche insbesondere gestaucht wird), sodass insgesamt auch eine Scherkraft auf den Cantilever wirkt. Bei einem (hypothetischen) homogenen Cantilever wären die Dehnung an der Oberseite gleich groß wie die Stauchung an der Unterseite. Bei einem asymmetrischen Aufbau des Cantilevers durch das vorgeschlagene Schichtensystem hat die Dehnung an der Oberseite einen anderen Wert als die Stauchung an der Unterseite.

Die oben genannten Kräfte basieren alle auf dem sogenannten Elastizitätsmodul des Cantilevers. Das Elastizitätsmodul des Cantilevers ist eine Materialkonstante, die spezifisch für das verwendete Material des Cantilevers ist. Durch Wahl des Materials oder der Materialkomposition beziehungsweise durch Bearbeitung des Materials lässt sich das Elastizitätsmodul in einem gewissen Rahmen einstellen, so dass der zu messende Effekt für die jeweils eingerichteten Transducer optimiert werden kann. Umgekehrt ist es natürlich auch möglich die Transducer auf das vorliegende Elastizitätsmodul des Materials anzupassen und deren Empfindlichkeit zu optimieren.

Bevorzugt können die Cantilever mehrschichtige Cantilever sein, beispielsweise Cantilever aus einer Gold- und einer Siliziumnitridschicht sowie einer Funktionsschicht auf der Goldschicht. Ein so gearteter Cantilever besteht aus Materialschichten die zusammen einen definierten Spannungszustand aufweisen. Beispielsweise kann der Zustand spannungsfrei sein, so dass die intrinsischen mechanischen Spannungen minimal sind. Es kann aber auch sein, dass ein der mehrschichtige Cantilever vorgespannt ist, so dass der Cantilever besonders sensitiv auf eine Änderung der Oberflächenspannung reagiert. Es kann zudem auch sein, dass ein homogener Cantilever auf der Oberseite und der Unterseite unterschiedlich beschichtet wird, um den beschriebenen Effekt durch mehrschichtige Cantilever zu erhalten.

Durch Vergleich der durch die Transducer detektierten Verformungen und/oder Kräfte, kann auf eine durch die selektive Aufnahme des Analyten verursachten Einwirkung auf den Testcantilever und somit auf dessen Vorkommen, geschlossen werden. Bevorzugt kann auf die Größe des Vorkommens geschlossen werden.

Insbesondere detektieren die passiven Transducer auf den Basen der Cantilever lediglich Effekte, die primär keine Auslenkung sind, da die Basen fest an das Substrat gekoppelt sind. Die Messsignale der passiven Transducer ergeben somit ein Grundsignal, welches spezifisch für den jeweiligen Cantilever ist.

Beispielsweise kann die Basis des Referenzcantilevers durch Einfluss der Umgebungsbedingungen einen ersten elektrischen Zustand des passiven Referenztransducers hervorrufen, während die Wechselwirkung des Testcantilevers mit der Probe einen zweiten elektrischen Zustand des passiven Testtransducers hervorruft.

Die aktiven Transducer auf den verformbaren Teilen der Cantilever hingegen geben ein Maß für die Verformung oder wirkende Kraft an und somit auch indirekt ein Maß für die Wechselwirkung der Referenzschicht beziehungsweise Rezeptorschicht mit dem Analyten und den anderen Stoffen der Probe.

Beispielsweise kann der Referenzcantilever durch Wechselwirkung mit der Probe um einen ersten Betrag verbogen werden, sodass die Auslenkung in dem aktiven Referenztransducer einen dritten elektrischen Zustand hervorruft, wohingegen der Testcantilever durch Wechselwirkung mit der Probe um einen zweiten Betrag verbogen wird und durch zusätzliche Wechselwirkung mit dem Analyten in der Probe um einen dritten Betrag verbogen wird, was im aktiven Testtransducer einen vierten elektrischen Zustand hervorruft.

Der Vergleich der elektrischen Zustände der passiven und aktiven Transducer geben ein Maß für die Verformung der Cantilever an, wobei das elektrische Signal der aktiven Transducer auf das Grundsignal der passiven Transducer auf der Basis korrigiert wird. Gleichzeitig ergibt ein Vergleich der Messsignale der aktiven Transducer ein Maß für die Unterschiedlichkeit der Verformung der Cantilever. Dadurch ist es möglich auf einen spezifischen Einfluss eines Analyten auf den Testcantilever zu schließen.

Die Bauweise mit vier Transducern hat den Vorteil, dass eine solche lokale Korrektur des Sensors am Ort des Einflusses der Probe und des Analyten möglich ist.

Die verformbaren Teile der Referenz- und Testcantilever können identische geometrische Abmessungen aufweisen, wobei bevorzugt die Breite des verformbaren Teils der Referenz- und Testcantilever der Länge des verformbaren Teils der Referenz- und Testcantilever entspricht.

Dadurch kann eine besonders große Kraft durch die Verformung der Cantilever auf die aktiven Transducer erzeugt werden.

Die Basen des Referenz- und Testcantilevers können auf derselben Gesamtbasis angeordnet sein. Insbesondere weisen der Testcantilever und der Referenzcantilever eine gemeinsame Basis auf.

Dadurch kann erreicht werden, dass die passiven Transducer auf einem ähnlichen Grundniveau arbeiten, beziehungsweise dass Einflüsse, die für die getrennten Basen spezifisch wären, reduziert sind. Dies kann zu einer größeren Messgenauigkeit führen.

Insbesondere können die Referenz- und Testcantilever dadurch besonders nahe beieinander angeordnet sein, beispielsweise kleiner als die Breite eines Cantilever.

Dadurch kann erreicht werden, dass auf die Cantilever identische Störeinflüsse wirken, die beispielsweise durch einen Temperaturunterschied in der Probe, insbesondere durch Konvektion oder eine andere Fluiddynamik verursacht wird.

Des Weiteren kann dadurch auch erreicht werden, dass mehrere Cantilever aus einem (Silizum-) Wafer hergestellt werden können.

Des Weiteren kann der Abstand der Cantilever hin zur Herstellungsgrenze optimiert werden. Die Herstellungsgrenze ist typischerweise durch den Spotting-Abstand gegeben, wobei der Spotting-Abstand ein Maß ist, welches beim Herstellen der Referenz und Rezeptorschicht relevant ist, siehe unten.

Die Basen des Referenz- und Testcantilevers können einteilig ausgebildet sein.

Dadurch kann erreicht werden, dass die Basis-spezifischen Einflüsse weiter reduziert werden, sodass eine größere Messgenauigkeit erreicht wird. Des Weiteren kann dadurch eine einfachere Herstellung des Cantileverpaars oder einer Vielzahl von Cantileverpaaren ermöglicht werden Insbesondere können die verformbaren Teile der Referenz- und Testcantilever also identische geometrische Abmessungen aufweisen, wobei die Breite der verformbaren Teile der Referenz- und Testcantilever der Länge der verformbaren Teile der Referenz- und Testcantilever entspricht, die Basen der Referenz- und Testcantilever auf derselben Gesamtbasis angeordnet sind und die Basen bevorzugt einteilig ausgebildet sind.

Die Referenz- und Testcantilever können Si3N4, SiO2, Si3N4/SiO2, SiC, Si umfassen oder aus Si bestehen oder ein Polymer umfassen.

Durch die siliziumbasierten Referenz- und Testcantilever lassen sich aus der Halbleiterindustrie bekannte Herstellungsverfahren verwenden, sodass die Herstellung der vorgeschlagenen Sensoren in einem großen industriellen Maßstab ermöglicht wird. Polymere lassen sich ebenfalls in großem industriellem Maßstab herstellen und weisen den Vorteil auf, dass deren Materialeigenschaften in großem Umfang vorbestimmt werden können.

Die Transducer können identische intrinsische physikalische Eigenschaften aufweisen, wobei die Transducer dazu eingerichtet sind ihren elektrischen Eigenschaften, bevorzugt den elektrischen Widerstand oder einen anderen zum k-Wert proportionalen Wert (siehe Gleichung oben), entsprechend den auf die Referenz- und Testcantilever wirkenden Kräfte anzupassen.

Identische intrinsische physikalische Eigenschaften umfassen hierbei diejenigen Eigenschaften, die für die Messeigenschaften des Transducers auf einem Cantilever verantwortlich sind. Dies betrifft insbesondere eine Spannung die über dem Transducer abfallen kann, sprich den Widerstand beziehungsweise die Leitfähigkeit des Transducers. Der Widerstand hängt insbesondere von der Geometrie des Transducers ab, sodass bei einer gleichförmigen Leitfähigkeit der verschiedenen Transducer dementsprechend die Geometrie der Transducer gleich sein muss. Eine ungleichförmige Leitfähigkeit der verschiedenen Transducer kann aber durch veränderte nanoskopische Materialeigenschaften der Transducermaterialien erzeugt werden.

Die physikalischen Eigenschaften betreffen aber auch die Art und Weise der Messsignaländerung als Reaktion auf eine wirkende Kraft. Insbesondere soll jeder Transducer gleich auf eine gleiche Krafteinwirkung beziehungsweise Verformung des Cantilevers reagieren, sodass zwischen den verschiedenen Transducer keine nichtlinearen Abweichungen auftreten können.

Die intrinsischen physikalischen Eigenschaften werden insbesondere durch die Nanostruktur der Transducer bestimmt. Die Nanostrukturen sind bevorzugt für alle Transducer identisch, so dass identische geometrische Konfigurationen identische physikalische Eigenschaften liefern.

Eine besonders hohe Sensitivität kann insbesondere dadurch erreicht werden, dass die Metallpartikelkorngröße der Transducer möglichst klein ist.

Durch einen zuverlässigen Herstellungsprozess der Transducer kann somit gewährleistet werden, dass alle Transducer gleich auf eine Kraft oder einen externen Störeinfluss reagieren, sodass Abweichungen der verschiedenen gemessenen Kräfte lediglich auf der äußeren Einwirkung auf die Cantilever beruhen und nicht von den intrinsischen physikalischen Eigenschaften abhängen.

Insbesondere kann über die elektrischen Eigenschaften der Transducer schließlich auf die Biegezustände der individuellen Referenz- und Testcantilever geschlossen werden, wobei insbesondere auf das Vorkommen des durch die Rezeptorschicht selektiv aufgenommenen Analyten geschlossen werden kann. Die Transducer, die über den Cantilever indirekt eine Wechselwirkung mit dem Analyten wahrnehmen variieren ihrer Messeigenschaften den einwirkenden Kräften entsprechend.

Der Abstand des aktiven Referenztransducers beziehungsweise Testtransducers und des passiven Referenztransducers beziehungsweise Testtransducers kann kleiner als 100 $\mu$m groß sein, wobei die Transducer an der Biegekante anliegen können.

Indem die Transducer möglichst nahe beieinander platziert werden, werden räumliche Einflüsse auf die Transducer die von der Probe herrühren verringert. Wenn beispielsweise das Vorkommen des

Analyten in der Probe einem gewissen Konzentrationsgradienten unterliegt, ist es vorteilhaft die Messungen an möglichst einem Punkt des Gradienten durchzuführen.

Der kleinstmögliche Abstand ist erreicht, wenn die Transducer an der Biegekante anliegen. Die Biegekante ist hierbei die Kante des Substrats entlang derer der Cantilever in die Basis und den verformbaren Teil unterteilt wird. Beispielsweise können die aktiven Transducer mit ihrer Unterkante an der Biegekante anliegen, während die passiven Transducer mit ihrer Oberkante an der Biegekante anliegen können.

Insbesondere kann der optimale Abstand der aktiven Transducer zur Biegekante von der genauen geometrischen Ausformung des Sensors abhängen. Dementsprechend kann der Abstand zur Biegekante so gewählt werden, dass eine Oberflächendehnung eine maximale Änderung des elektronischen Zustands des Transducers erzeugt.

Insbesondere ist der optimale Abstand der passiven Transducer zur Biegekante erreicht, wenn durch die Auslenkung Testcantilevers eine möglichst geringe Änderung des elektronischen Zustands des passiven Transducers erreicht wird.

Insbesondere sollten die aktiven und passiven Transducer jedoch so nah beieinander angeordnet sein, dass sie in einem Herstellungsprozess, beispielsweise in einem Rasterelektronenmikroskopbasierten oder elektronenlithographischen Herstellungsprozess einfach und schnell in einem Schritt zusammen hergestellt werden können, ohne dass eine mechanische Bewegung einer XYZ-Vorschiebevorrichtung den Wafer verschieben muss. Dadurch kann insbesondere eine deutlich schnellere und genauere sowie eine kostengünstige Herstellung der Sensoren ermöglicht werden.

Insbesondere bestimmt die Orientierung und Geometrie der Transducer ob eine Längs-, eine Querdehnung, oder eine Mischdehnung der Cantilever gemessen wird. Wenn eine Längsachse des Transducers parallel zur Basis verläuft, wird bevorzugt eine Querdehnung des Cantilevers gemessen. Wenn eine Längsachse des Transducers senkrecht zur Basis orientiert ist, wird bevorzugt die Längsdehnung des Cantilevers gemessen. Die Längsachse eines Transducers kann aber auch eine beliebige Orientierung bezüglich der Basis aufweisen und entsprechend eine Mischdehnung (eine Überlagerung einer Läng- und einer Querdehnung) messen. Es ist daher insbesondere auch möglich rechteckige, quadratische, runde, konvexe oder konkave Transducer zu formen, um die Empfindlichkeit des Transducers an die Cantilevergeometrie anzupassen.

Die Referenz- und Testcantilever, sowie die aktiven und passiven Referenz- und Testtransducer können spiegelsymmetrisch zueinander angeordnet sein.

Die Spiegelsymmetrie kann sich insbesondere auf eine Spiegelachse beziehen, die zwischen dem Referenz- und dem Testtransducer angeordnet ist.

Durch einen spiegelsymmetrischen Aufbau ist es möglich, dass Einflüsse beispielsweise von elektrischen Spannungen auf die Transducer reduziert werden, oder zumindest symmetrisch zueinander geführt werden. Dadurch kann die Messgenauigkeit und Störanfälligkeit verbessert werden.

Die Test-Wandlerschicht und Referenz-Wandlerschicht des Sensors kann Elektroden umfassen, bevorzugt vier Elektroden, die dazu eingerichtet sind die aktiven und/oder passiven Testtransducer sowie die passiven und/oder aktiven Referenztransducer elektrisch zu kontaktieren.

Eine Elektrode ist hierbei eine leitfähige Schicht, beispielsweise aus Gold, oder ein Draht beziehungsweise Kabel, welche von einem Anschlussende des Transducers eine elektrisch leitfähige Verbindung zu einem externen Gerät, wie beispielsweise einer Strom- oder Spannungsquelle beziehungsweise zu einem entsprechenden Messgerät herstellen kann. Prinzipiell kann jede leitfähige Verbindung zwischen dem Transducer und dem externen Gerät als Elektrode verstanden werden. Jedoch wird hier als Elektrode insbesondere der Teil der elektrischen Verbindung angesehen, die den Sensor kontaktiert.

Typischerweise wird eine elektrische Verbindung vom Sensor zu einer externen Quelle oder einem Messgerät über einen elektrischen Anschluss realisiert. Hierbei wird ein elektrischer Anschlussstecker mit einem Kabel oder einem Draht auf ein sogenanntes Bondpad kontaktiert, beispielsweise in dem der Draht dort mit Ultraschall fest geschweißt wird. Von dem Bondpad führt anschließend eine elektrische Verbindung direkt zum Transducer. Die elektrische Isopotentialfläche zwischen Transducer und Bondpad wird im Folgenden Elektrode genannt.

Die Elektrode dient dazu die Transducer elektrisch zu kontaktieren und insbesondere die Möglichkeit zu schaffen die elektrischen Signale von dem Sensor an ein Messgerät zu führen.

Insbesondere können die Elektroden auf unterschiedlichen elektrischen Potenzialen liegen und über diese miteinander in Wechselwirkung treten. Um diese gegenseitige Beeinflussung der elektrischen Ströme und Spannungen in den Elektroden zu minimieren ist es daher vorteilhaft, wenn die Elektroden ebenfalls eine symmetrische Form aufweisen, sodass die jeweilige Störung zumindest gleichförmig auf das Gesamtsystem verteilt wird. Dies kann insbesondere dadurch realisiert werden, dass eine gerade Anzahl von Elektroden verwendet wird beziehungsweise dass bei vier Transducer lediglich vier Elektroden verwendet werden.

Durch die Auslegung der Elektrodengeometrie ist es daher möglich, dass der Grundsignalpegel, der sich an den Elektroden durch etwaige Potenzialdifferenzen ergibt, kleiner als 1,1 V ist, sodass eine elektrische Verkapselung der Elektroden nicht notwendig ist. Unter elektrischer Verkapselung kann hierbei beispielsweise eine elektrische Isolation oder Abdeckung oder Abschirmung der Elektroden und der Bonddrähte verstanden werden. Dadurch kann der Herstellungsprozess vereinfacht werden und die Messgenauigkeit wird verbessert.

Der Abstand zwischen den Elektroden kann minimal sein.

Minimal ist hierbei der Abstand, wenn sich die Elektroden nicht berühren, sprich nicht leitfähig miteinander verbunden sind. Mit anderen Worten ist der Leitwert zwischen den Elektroden wesentlich geringer als der Leitwert der Transducer.

Indem der Abstand zwischen den Elektroden minimal ist, kann die Zahl der Elektroden auf einem Wafer platziert werden, sodass ein kosteneffizienter Herstellungsprozess möglich wird. Insbesondere kann dadurch aber auch die Größe der Transducer reduziert werden, so dass der Einfluss ungleichförmiger Umgebungsbedingungen auf die Transducer weiter reduziert werden kann.

Die Transducer können in einer Vollbrücke elektrisch verschaltet sein, wobei die Vollbrücke dazu eingerichtet ist, aufgrund der elektrischen Eigenschaften der Transducer, insbesondere bei einer asymmetrischen Änderung der elektrischen Eigenschaften der Transducer, eine Brückenquerspannung aufzubauen.

Eine Vollbrücke ist hierbei eine Messeinrichtung zur Messung von elektrischen Widerständen beziehungsweise von kleinen Widerstandsänderungen. Eine Vollbrücke ist auch bekannt unter den Bezeichnungen Wheatstone'sche Messbrücke oder H-Brücke oder symmetrische Vollbrücke oder thermisch-symmetrische Vollbrücke.

Beispielsweise werden die aktiven und passiven Transducer der Referenz- und Testcantilever zu einer Vollbrücke verschaltet, in dem je ein Anschlusskontakt der aktiven Transducer über eine erste Elektrode auf ein gemeinsames Potenzial gelegt wird. Des Weiteren wird je ein Anschlusskontakt der passiven Transducer über eine dritte Elektrode auf ein gemeinsames Potenziale gelegt. Über diese erste und dritte Elektrode kann eine Spannung (Gleich- oder Wechselspannung) angelegt werden, wobei der Zusammenschluss der aktiven Transducer beziehungsweise der passiven Transducer je als Spannungsteiler entsprechend den Widerständen der jeweiligen Transducer wirkt.

Zudem werden auf jedem Cantilever der weitere Anschlusskontakt des aktiven Transducers mit dem weiteren Anschlusskontakt des passiven Transducers über eine zweite Elektrode beim Testcantilever beziehungsweise vierte Elektrode beim Referenzcantilever miteinander verbunden. Über der zweiten und vierten Elektrode baut sich dementsprechend eine Brückenquerspannung auf, sofern das Verhältnis der Widerstände des aktiven Transducers zum passiven Transducer des Referenzcantilevers ungleich dem Verhältnis der Widerstände des aktiven Transducers zum passiven Transducer des Testcantilevers ist.

Im Grundzustand der Vollbrücke des Sensors ist die Brückenquerspannung idealerweise gleich null, da auf alle beteiligten Transducer keine Kraft beziehungsweise eine gleiche Kraft wirkt. Dieser Grundzustand wird vorzugsweise bereits beim Herstellungsprozess eingestellt, sodass sich zwischen den Elektroden nur eine geringe Offsetspannung einstellt, die über einen messtechnischen Aufbau kompensiert werden kann.

Von diesem Grundzustand der Vollbrücke aus lassen sich dann bevorzugt asymmetrische Kraftänderungen detektieren. Wenn beispielsweise der aktive Testtransducer des Testcantilevers auf eine Krafteinwirkung mit einer Änderung seiner elektrischen Eigenschaft respektive mit Änderung seines elektrischen Widerstandes reagiert, dann ist das Verhältnis der Widerstände in der Vollbrücke nicht mehr ausgeglichen, sodass sich eine Brückenquerspannung aufbaut. Die aufgebaute Brückenquerspannung kann schließlich mit einem Messgerät detektiert werden.

Insbesondere baut sich keine Brückenquerspannung auf, wenn die Krafteinwirkung auf die aktiven Transducer des Testcantilevers und des Referenzcantilevers gleich ist. Dies ist dann jedoch eine unspezifische Krafteinwirkung, die nicht von einer spezifischen Wechselwirkung mit dem Testcantilever stammt. Insbesondere baut sich auch keine Brückenquerspannung auf, wenn die Krafteinwirkung auf die passiven Transducer des Testcantilevers und des Referenzcantilevers gleich ist.

Über die Realisierung als Vollbrücke wird über den aktiven Referenztransducer des Referenzcantilevers quasi eine Kalibrierung des aktiven Testtransducers des Testcantilevers herbeigeführt. Durch die passiven Transducer wird einerseits eine Kalibrierung auf den

Grundzustand der Vollbrücke ermöglicht, zum anderen kann durch Vergleich der aktiven und passiven Transducer auf eine Auslenkung der verformbaren Teile der Cantilever geschlossen werden.

Der Sensor kann einen Brückenquerspannungsdetektor umfassen, der dazu eingerichtet ist, die Brückenquerspannung der Vollbrücke zu detektieren, wobei durch die detektierte Brückenquerspannung auf das Vorkommen des durch die Rezeptorschicht selektiv aufgenommenen Analyten geschlossen wird, bevorzugt auf die Größe des Vorkommens.

Ein Brückenquerspannungsdetektor kann insbesondere jeder Detektor sein, der in der Lage ist eine Spannung zu detektieren. Beispielsweise kann das ein Messwiderstand sein, oder ein Signalgeber oder ein Messgerät, welches die Spannung anzeigt oder eine andere Art von Detektor, der durch die Detektion einer Spannung ein Ausgabesignal erzeugt.

Der Brückenquerspannungsdetektor kann dazu eingerichtet sein, einen einzigen Ausgabewert zu erzeugen, sodass lediglich das Vorkommen einer Brückenquerspannung angezeigt wird. Insbesondere kann aufgrund des Vorkommens eine Brückenquerspannung darauf geschlossen werden, dass ein Analyt in einer gewissen Mindestkonzentration mit der Rezeptorschicht des Testcantilevers in Wechselwirkung getreten ist, und dadurch die elektrischen Eigenschaften der Transducer, beziehungsweise mindestens die elektrische Eigenschaft des aktiven Transducers des Testcantilevers, verändert wurden.

Ein Brückenquerspannungsdetektor kann jedoch auch verschiedene Ausgabewerte anzeigen, die vorzugsweise in einem einfachen funktionalen Zusammenhang zur Brückenquerspannung stehen. Beispielsweise kann das bedeuten, dass der Ausgabewert des Brückenquerspannungsdetektors ansteigt, sofern die Brückenquerspannung ansteigt. Es kann aber auch bedeuten, dass der Ausgabewert des Brückenquerspannungsdetektors abfällt, sofern die Brückenquerspannung ansteigt. Besonders vorteilhaft ist es, wenn von dem Ausgabewert des Detektors auf einen eindeutigen Wert der Brückenquerspannung geschlossen werden kann. Mit anderen Worten ist es bevorzugt, wenn der Ausgabewert des Brückenquerspannungsdetektors einer bijektiven Funktion der Brückenquerspannung folgt.

Idealerweise wird die Änderung der Brückenspannung als ratiometrische Änderung in Bezug auf eine definierte, sprich gemessene Versorgungsspannung ausgedrückt. Beispielsweise beeinflusst dann eine Drift in der Versorgungsspannung das Messsignal nicht.

Die verschiedenen Ausgabewert müssen sich nicht auf die Amplitude des Signals beschränken, sondern können sich auch auf das zeitliche Auftreten der Ausgabewert beschränken. Beispielsweise kann der Brückenquerspannungsdetektor bei einer ersten Spannung einen Puls pro Zeitintervall abgeben, während einer zweiten Spannung der Brückenquerspannungsdetektor viele Pulse pro Zeitintervall abgibt. Somit kann durch das Auftreten der Pulse die Stärke der Brückenquerspannung angezeigt werden. Insbesondere kann somit der Ausgabewert kodiert werden.

Die elektrischen Eigenschaften der Transducer können über eine AD-Wandler ausgegeben werden und eine AD-Wandlerlogik kann dazu eingerichtet sein eine differentielle Messung und/oder eine absolute Messung der Biegezustände zur Verfügung zu stellen.

Insbesondere kann der Brückenquerspannungsdetektor in Form eines AD-Wandlers ausgestaltet sein, wobei ein AD-Wandler eine Wandlerelektronik ist, die aus einem analogen Signal ein digitales Signal generiert. Hierfür wird beispielsweise die Stärke des Messsignals punktweise mit einer gewissen Periodizität von dem AD-Wandler abgetastet, und die gemessene Spannung in einen Digitalwert übersetzt.

Der AD-Wandler kann insbesondere eine AD-Wandlerlogik umfassen, wobei die AD-Wandlerlogik durch Anpassung der inneren Verschaltung, insbesondere durch Softwareanpassungen in verschiedenen Betriebsmodi versetzt werden kann. Über die verschiedenen Betriebsmodi können verschiedene Spannungen (insbesondere Wechselspannungen und/oder Gleichspannungen) und Messsignale von der Elektrodenschaltung abgegriffen werden.

Beispielsweise kann der AD-Wandler einen sogenannten differenziellen Messmodus aufweisen, bei dem lediglich die Biegezustandsänderung zwischen dem Referenzcantilever und dem Testcantilever erfasst wird. In diesem differenziellen Messmodus wird insbesondere die Brückenquerspannung abgegriffen, sodass eine Änderung der Biegezustände der Cantilever in Form einer auftretenden Brückenquerspannung detektiert wird. Der differentielle Messmodus ist der bevorzugte Messmodus zum Detektieren einer Bindung eines Analyten an die Rezeptorschicht.

Es ist jedoch auch möglich den AD-Wandler in einem sogenannten absoluten Messmodus zu betreiben bei dem über die Elektroden direkt auf die einzelnen Transducer zugegriffen wird (Single-Ends-Modus). Dies erlaubt das Messen der beiden Halbbrücken, beispielsweise zur Qualitätssicherung, oder auch zur Charakterisierung der Vollbrücke. Des Weiteren ist es darüber möglich die absoluten Biegezustände der Cantilever zu detektieren.

Insgesamt kann also ein AD-Wandler die Brückenquerspannung in ein digitales Signal umwandeln, wobei der AD-Wandler über eine AD-Wandlerlogik in einem differentiellen Messmodus und/oder in einem absoluten Messmodus betrieben werden kann.

Insbesondere wird bei dieser Bauweise lediglich ein AD-Wandler benötigt, sodass der Herstellungsprozess kostengünstig realisiert werden kann.

Zudem ist es wegen der stabilen und ausgeglichenen Vollbrücke auch möglich den AD-Wandler weit von den eigentlichen Transducer und Cantilevern zu entfernen, sodass beispielsweise eine Abwärme des AD-Wandlers das Messergebnis nicht beeinflusst.

Der Sensor kann auf einem Chip ausgebildet sein.

Dies kann bedeuten, dass der Sensor auf einer Halbleiterstruktur hergestellt wird, die weitergehende Datenverarbeitung der Brückenquerspannung beziehungsweise der Ausgabewert der AD-Wandlerlogik zulässt. Insbesondere kann mit Chip auch ein sogenanntes System-On-A-Chip gemeint sein, wobei alle funktionalen Einheiten des Messsystems integral auf einem einzigen elektronischen Bauteil ausgebildet werden.

Dabei ist jedoch zu berücksichtigen, dass die Prozesskette der Herstellung des Sensors Gold umfassen kann, was die Herstellung einer AD-Wandlerlogik durch CMOS Halbleitertechniken beeinträchtigen kann.

Eine Vielzahl von Cantileverpaaren kann auf einem Chip angeordnet werden, wobei eine AD-Wandlerlogik dazu eingerichtet sein kann ein Signalmultiplexing der Messsignale zur Verfügung zu stellen.

Ein Cantileverpaar umfasst jeweils einen Referenzcantilever und einen Testcantilever. Auf einem Chip kann eine Vielzahl von solchen Cantileverpaaren mitsamt aktiver und passiver Transducer angeordnet sein, die jeweils wiederum über eine AD-Wandlerlogik ausgelesen werden können.

Es kann insbesondere auch sein, dass ein erstes Cantileverpaar spezifisch auf einen ersten Analyten reagiert und ein zweites Cantileverpaar auf einen zweiten Analyten reagiert, so dass mit einem Sensor verschiedene Analyten detektiert werden können.

Eine Vielzahl von Cantileverpaaren kann jedoch auch eine erste Anzahl von Testcantilevern umfassen und eine zweite Anzahl von Referenzcantilevern umfassen. Beispielsweise können die verschiedenen Referenzcantilever verschiedene Störeinflüsse besonders sensitiv detektieren, die zusammen die Referenz für die Anzahl an Testcantilevern liefert.

Über eine entsprechende AD-Wandlerlogik lassen sich die Cantileverpaare gleichzeitig betreiben. Dadurch ist zum einen das Detektieren vieler verschiedener Analyten mittels verschiedener Rezeptor und Referenzschichten möglich. Zum anderen ist es aber auch möglich, durch gleiche Rezeptorschichten und Referenzschichten eine statistische Aussage über die Signifikanz der gemessenen Brückenquerspannungen zu etablieren.

Die Test-Wandlerschicht und/oder Referenz-Wandlerschicht auf den oberen Oberflächen der Test- und/oder Referenzcantilever können eine Aktivierungsschicht umfassen, wobei die Aktivierungsschicht dazu eingerichtet ist, im Falle einer Krafteinwirkung auf den Referenz- und Testcantilever, eine im Vergleich zur nichtaktivierten unteren Oberfläche des Referenz- und Testcantilevers, eine größere Oberflächendehnung zur Verfügung zu stellen, und wobei die Aktivierungsschicht beispielsweise aus Gold besteht.

Eine Aktivierung der oberen Oberfläche kann bedeuten, dass durch Aufbringen einer Aktivierungsschicht eine Haftvermittlung für eine weitere Schicht zur Verfügung gestellt wird. Dies kann darin begründet sein, dass das Basismaterial des Cantilevers beispielsweise keine Bindung mit einer Folgeschicht eingeht. Die Folgeschicht kann dabei, wie weiter unten beschrieben, eine Funktionsschicht sein.

Insbesondere kann die Aktivierungsschicht Gold umfassen, oder ganz aus Gold bestehen.

Indem die obere Oberfläche eine Aktivierungsschicht aufweist, ist insbesondere der Aufbau der Cantilever in der Höhe nicht homogen beziehungsweise asymmetrisch, sondern besteht aus Schichten. Dadurch kann die Elastizität bzw. Steifigkeit des Cantilevers maßgeblich beeinflusst werden, sodass an der oberen Oberfläche bei einer Verformung der Cantilever eine größere Oberflächendehnung entsteht, die wiederum zu einem größeren Messsignal führt.

Die Beschichtung der Cantilever mit Gold kann aufgrund der guten Leitfähigkeit ebenfalls dazu verwendet werden, Elektroden für die Transducer auszubilden. Aus diesem Grund kann auch der Abstand zwischen den Elektroden minimiert werden, da so möglichst wenig Fläche des Cantilevers nicht mit Gold belegt wird. Dementsprechend kann die beschichtete Fläche maximiert werden, wodurch sich eine besonders große Verformung ergibt.

Die Aktivierungsschicht kann insbesondere auch aus einer Chrom-Gold-Legierung bestehen, da dadurch die mechanischen Eigenschaften des Cantilevers weniger beeinflusst werden. Insbesondere wird durch die Beimischung von Chrom eine Homogenität der Kristallite der Goldschicht erreicht, so dass eventuell störende Anisotropieeffekte durch das Kristallgitter einer hypothetischen kristallinen Schicht vermieden werden können.

Die unteren Oberflächen der Referenz- und Testcantilever können durch eine Passivierungsschicht passiviert sein, wobei die Passivierungsschicht dazu eingerichtet ist eine unspezifische Proteinadhäsion auf den Referenz- und Testcantilever zu minimieren, und wobei die Passivierungsschicht Trimethoxisilan und/oder eine Blocking-Substanz umfasst.

Im Unterschied zu einer Aktivierungsschicht ist eine Passivierungsschicht eine Schicht, die eine Wechselwirkung zwischen dem Cantilever und einem anderen Material minimieren oder unterbinden soll. Dies hat zur Folge, dass bei der Herstellung der Rezeptorschicht bzw. Referenzschicht diese lediglich an der oberen Oberfläche des Cantilevers bindet und nicht an der unteren Oberfläche des Cantilevers bindet. Dadurch kann durch Bindung der Rezeptorschicht bzw. Referenzschicht mit einem Analyten eine größere Oberflächenspannung an der oberen Oberfläche erreicht werden. Weiter wird dadurch die Asymmetrie des Schichtaufbaus verstärkt, was zu verbesserten Dehnungseigenschaften für die Signaldetektion führen kann.

Insbesondere eignen sich für die Passivierung der unteren Oberfläche die Materialien Trimethoxisilan, sowie sogenannte Blocking-Schichten. Durch diese Passivierungsschicht wird eine sogenannte unspezifische Proteinadhäsion minimiert. Die Proteinadhäsion ist eine Adhäsion eines Proteins an der Oberfläche. Eine unspezifische Adhäsion eines Proteins oder eines Stoffes im Allgemeinen, an den Cantilever kann zu Verzerrungen des Messergebnisses führen, da diese unspezifischen Stoffe ebenfalls mit den Cantilever in Wechselwirkung treten. In dem diese unspezifische Adhäsion unterbunden wird, vergrößert sich der relative Einfluss der gewollten spezifischen Adhäsion oder Wechselwirkung des Analyten mit dem Cantilever relativ zum Grundzustand des Cantilevers.

Es ist aber auch möglich, dass eine Passivierungsschicht auch den Analyten bindet, jedoch in einer Art und Weise, dass die daraus resultierende Oberflächenspannung der Oberflächenspannung der Aktivierungsschicht entgegengesetzt ist. Dadurch kann eine größere Verformung der Cantilever erreicht werden

Die sogenannte Blocking-Schicht kann insbesondere auf den jeweils untersuchenden Analyten angepasst werden, um ein Messfenster für den Analyten zu definieren. Die Blocking-Schicht wird dabei im sogenannten Spottingprozess oder Waschprozess aufgebracht.

Beim Waschprozess schützt ein sogenannter "Sealer" beim Eintrocknen die Hydrathülle der Detektorproteine und macht diese somit lagerfähig. Der Sealer ist löslich in einer Matrix eingebunden, so dass er löslich für eine Probenflüssigkeit wie Wasser ist. Zudem weist der Sealer eine gewisse Schichtdicke auf, so dass die Cantilever mechanisch stabilisiert werden, was den Schutz bei der Lagerung der Cantilever vergrößert. Ein Sealer kann beispielsweise Zucker enthalten. Die Zuckerkristalle sind hydrophil und schützen daher die Hydrathülle der Proteine. Somit ist eine sogenannte Rekonstituierung der Proteine, bei der die getrockneten Proteine in der Messflüssigkeit wieder aktiviert werden, möglich.

Beim Spotting der Rezeptorproteine werden sogenannte "Puffer" genutzt, um eine Rekonsitituierung der Proteine in der Probenflüssigkeit zu ermöglichen. Auch hier wird durch ein Eintrocknen die Lagerfähigkeit der Sensoren erhöht.

Der aktive und der passive Cantilever können chemisch identisch aufgebaut sein.

Hierzu weisen der Referenzcantilever und der Testcantilever einen identischen Schichtaufbau auf, der sich lediglich darin unterscheidet, dass auf dem Testcantilever eine Rezeptorschicht aufgebracht ist und auf dem Referenzcantilever eine Referenzschicht. Insbesondere meint somit die chemische Identität, dass sich die beiden Cantilever nur in der Referenz- beziehungsweise Testschicht unterscheiden.

Dadurch wird erreicht, dass das Messsignal, insbesondere bei einer differentiellen Messung der Brückenquerspannung, lediglich auf dem Einfluss des Analyten auf die Cantilever basiert und nicht durch weitere Eigenschaften der Cantilever hervorgerufen wird.

Insbesondere bezieht sich die chemische Identität darauf, dass die Cantilever insofern verändert und angepasst werden, als dass sie sich nur über ihre Bindungseigenschaften beziehungsweise Wechselwirkungseigenschaften an den zu messenden Analyten unterscheiden. Für alle weiteren Stoffe soll eine möglichst gleiche Wechselwirkung, beziehungsweise eine möglichst geringe Wechselwirkung erreicht werden.

Insgesamt kann der oben beschriebene gesamte Schichtaufbau der Cantilever auch invertiert werden. Das bedeutet, dass die Referenz- und Rezeptorschichten anstatt auf der oberen

Oberfläche auch auf der unteren Oberfläche der Cantilever aufgebracht werden können. Beispielsweise kann die Rezeptorschicht auch auf der Unterseite des Cantileversangeordnet werden.

Damit sich der Cantilever verformt, sollte idealerweise jegliche chemische Anbindung an den Cantilever einseitig geschehen. Wenn der Analyt auf der Oberseite bindet, sollte auf der Unterseite des Cantilevers keine unspezifische Bindung geschehen, da sonst die Oberflächenspannung, die aus der chemischen Bindung des Analyten resultiert durch die unspezifische chemische Bindung auf der Unterseite des Cantilevers kompensiert werden kann.

Mit anderen Worten muss die chemische Anbindung auf der Oberseite und der Unterseite zumindest asymmetrisch sein, um eine Verformung zu erreichen. Eine stärkere Anbindung auf der Oberseite als auf der Unterseite oder eine stärkere Anbindung auf der Unterseite als auf der Oberseite führt entsprechend zu einer messbaren Verformung des Cantilevers.

Die Rezeptorschicht kann Antikörper für ein Antigen umfassen und die Referenzschicht kann einen auf den Antikörper der Referenzschicht ausgerichteten antigen-spezifischen Isotypkontroll-Antikörper umfassen.

Antikörper sind Proteine die von Körperzellen als Reaktionsprodukt auf Antigene produziert werden. Antikörper werden typischerweise vom menschlichen Immunsystem dafür verwendet an die Antigene von z.B. Viren zu binden, sodass die Viren markiert und ein Ausbruch einer Virusinfektion durch das Immunsystem vermieden werden kann. Es kann insbesondere sein, dass ein Antikörper an verschiedene Antigene bindet, so dass die Spezifität des Antikörpers herabgesetzt ist. Antikörper können auch so ausgebildet sein, dass sie an nicht-immunologische Ziele binden, wie beispielsweise THC.

Ein Isotypkontroll-Antikörper bindet im Gegensatz dazu genau nicht an das Antigen eines Virus, sodass bei gleichzeitigem Vorliegen einer Bindung des Antikörpers an das Antigen und eines nicht bindendes des Isotypkontroll-Antikörpers an das Antigen mit einer hohen Spezifität das Vorliegen eines bestimmten Virus beziehungsweise eines Antigens eines Virus geschlossen werden kann.

Der Antikörper eines Antigens kann Teil der Rezeptorschicht des Testcantilevers sein, während der Isotypkontroll-Antikörper des Antigens Teil der Referenzschicht sein kann. Das hat den Vorteil, dass eine Auslenkung des Testcantilevers gleichzeitig durch eine Nichtauslenkung des Referenzcantilevers bestätigt werden kann.

Mit anderen Worten kann auf die unteren Oberflächen der Referenz- und Testcantilever eine Passivierungsschicht aufgebracht sein, auf die oberen Oberflächen der Referenz- und Testcantilever kann eine Aktivierungsschicht aufgebracht sein, auf die Aktivierungsschicht kann die Referenz- und Test-Funktionsschicht mit bevorzugt einer selbstorganisierenden Monoschicht aufgebracht sein, und bevorzugt auf die selbstorganisierende Monoschicht des Referenzbeziehungsweise Testcantilevers kann eine Referenz- beziehungsweise Rezeptorschicht aufgebracht sein, wobei die Rezeptorschicht Antikörper für ein Antigen umfasst und die Referenzschicht einen auf den Antikörper der Rezeptorschicht ausgerichteten antigenspezifischen Isotypkontroll-Antikörper umfasst.

Die Schichten können in einem Dipping/Spotting- Prozess hergestellt werden, wobei das Spotting bevorzugt mittels kommerziell verfügbarer Maschinen durchgeführt werden kann. Hierbei werden Tröpfchen der jeweiligen Schicht auf den Cantilever abgelegt, so dass eine räumliche Begrenzung der Funktionalisierung erreicht wird, was insbesondere eine kostengünstige und unabhängige Beschichtung der Cantilever ermöglicht. Die sehr kleinen Tropfen werden durch eine geeignete Kontrolle der Umgebungsparameter, wie Temperatur, Luftfeuchte und Taupunkt, am Trocknen gehindert. Die Unterseiten der Cantilever werden hierbei nicht aktiviert, sodass die verwendeten Antikörper lediglich mit der oberen Oberfläche des Cantilevers in Kontakt kommen. Anschließend werden die Schichten getrocknet, so dass eine erhöhte oder erniedrigte Temperatur wenig oder bevorzugt kein Einfluss auf die Antikörper hat. Dies erlaubt eine lange Lagerfähigkeit, insbesondere in einem Inertgas. Die Proteinschichten werden insbesondere nach dem Aufbringen der Funktionsschichten aber vor der Vereinzelung der Sensoren von dem Wafer aufgebracht.

Die Rezeptorschicht kann beispielsweise SARS-CoV2 Antikörper umfassen und die Referenzschicht SARS-CoV2-spezifische Isotypkontroll-Antikörper umfassen.Der Sars-Cov2-Antikörper bindet bevorzugt gegen das S1 oder N Antigen des Sars-CoV2 Virus. Der Antikörper ist monoklonal, sequenztreu und weist eine hohe Spezifität gegenüber dem Sars-CoV2-Antigen auf. Insbesondere kann der Antikörper durch die sogenannte Phagen-Display-Methode hergestellt werden. Der Sars-CoV2-spezifische Isotypkontroll-Antikörper hingegen kann ultrahochspezifisch gegen das entsprechende Antigen sein, aber sonst identisch zum aktiven Antikörper sein.

Dadurch ist es beispielsweise eine schnelle Detektion des Sars-CoV2-Virus möglich. Insbesondere ist durch die elektrische Messung und die Anlagerung der Antikörper an den Testcantilever ein schnelles Testverfahren gegeben, welches durch den Vergleich mit der nicht Anlagerung an dem Referenzcantilever zudem eine hohe Spezifität aufweist.

Die Rezeptorschicht kann im Allgemeinen molekülspezifische Bindungskräfte bereitstellen und die Referenzschicht molekülspezifische keine Bindungskräfte bereitstellt. Dadurch ist es möglich eine bestimmte Molekülspezies nachzuweisen.

Die Rezeptorschicht kann Einzelstrang-DNA (ssDNA) und/oder andere DNA-Fragmente umfassen, die spezifisch an DNA-Fragmente in der Probe binden kann. Die Referenzschicht kann Einzelstrang-DNA und/oder andere DNA-Fragmente umfassen, die an keine chemische und/oder biochemische und/oder physikalische Spezies in der Probe bindet, aber in charakteristischen Parametern (z.B. Kettenlänge, chemischer Aufbau) mit der Rezeptorschicht übereinstimmt.

Die Rezeptorschicht kann Einzelstrang-RNA und/oder andere RNA-Fragmente umfassen, die spezifisch an RNA-Fragmente in der Probe binden kann. Die Referenzschicht kann Einzelstrang-RNA und/oder andere RNA-Fragmente umfassen, die an keine chemische und/oder biochemische und/oder physikalische Spezies in der Probe bindet, aber in charakteristischen Parameter (z.B. Kettenlänge, chemischer Aufbau) mit der Rezeptorschicht übereinstimmt. Dadurch ist es möglich eine bestimmte DNA oder RNA sowie deren Fragmente und/oder andere Oligonukleotide nachzuweisen.

Die Rezeptorschicht kann Antikörper und/oder andere und/oder weitere Proteine umfassen, die Zielproteine spezifisch binden können und die Referenzschicht kann entsprechend spezifische Isotypkontroll-Antikörper und/oder weitere Proteine umfassen, die an keine chemische und/oder biochemische und/oder physikalische Spezies in der Probe binden.

Die Rezeptorschicht kann scFv-Antikörperteile umfassen und die Referenzschicht kann scFv-Antikörperteile -spezifische Isotypkontroll-Antikörper umfassen. Ein scFv-Antikörper ist ein künstlich hergestelltes Antikörperfragment. Indem ein Antikörper in mehrere Fragmente zerlegt werden, kann die Reaktivität des Sensors auf eine geringe Probenkonzentration gesteigert werden.

Die Rezeptorschicht und die Referenzschicht können Hydrogele umfassen.

Hydrogele sind molekulare Matrizen, die Wasser sehr gut binden können und die beim Kontakt mit Wasser stark anschwellen. Durch eine chemische Modifikation der Hydrogele, insbesondere der Matrix kann eine starke Reaktion des Hydrogels auf das Vorhandensein von Proteinen oder anderen Analyten bewerkstelligt werden, so dass die mechanische Verformung des Cantilevers vervielfacht wird. Insbesondere ist es so auch möglich eine pH-Wert-sensitive Messung des Analyten durchzuführen.

## Kurze Beschreibung der Figuren

Bevorzugte weitere Ausführungsformen der Erfindung werden durch die nachfolgende Beschreibung der Figuren näher erläutert. Dabei zeigen:

| | |
|---|---|
| Figur 1 | eine schematische Darstellung einer ersten Ausführungsform des Sensors; |
| Figur 2A, B, C, D | eine schematische Darstellung der Cantilever: |
| Figur 3A, B | eine schematische Darstellung einer zweiten Ausführungsform des Sensors; |
| Figur 4 | eine schematische Darstellung einer dritten Ausführungsform des |

Sensors;

Figur 5A, B, C      weitere schematische Darstellungen weitere Ausführungsformen des Sensors, sowie Schaltdiagramm einer Vollbrücke;

Figur 6      eine schematische Darstellung eines Chips mit mehreren Cantileverpaaren;

Figur 7      eine schematische Darstellung der Bindung von Antigenen an Antikörper;

Figur 8      Schematische Darstellung des Sensors in Form eines Cantilevers mit einer Funktionsschicht, welche die Sensorschicht abdeckt;

Figur 9A      Schematische Darstellung ausgewählter Schichten eines Cantilevers ohne Funktionsschicht mit eingezeichneter neutraler Achse;

Figur 9B      Schematische Darstellung ausgewählter Schichten eines Cantilevers mit zusätzlicher Funktionsschicht und einer nach unten verschobenen neutralen Achse;

Figur 10      Schematische Darstellung ausgewählter Schichten eines Cantilevers mit zusätzlicher strukturierter Funktionsschicht und einer am Ort der Strukturierung nach unten verschobenen neutralen Achse.

Figur 11A      Draufsicht auf die Wandlerschicht eines Cantilevers mit in der Ebene strukturierten Transducern

Figur 11B      Seitenansicht ausgewählter Schichten eines Cantilevers mit strukturiertem Höhenprofil der Transducer

Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

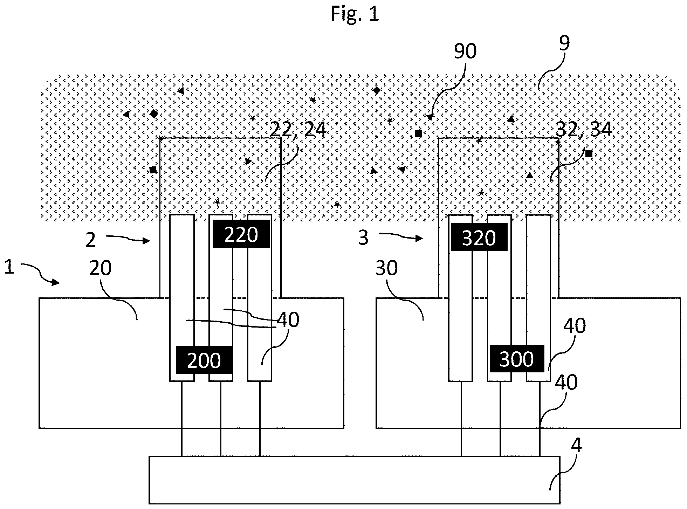

In Figur 1 ist schematisch eine erste Ausführungsform des vorgeschlagenen Sensors 1 zur Umwandlung chemischer und/oder biochemischer Information gezeigt. Der Sensor 1 umfasst einen Testcantilever 2, der wiederum eine Basis 20 aufweist, sowie einen verformbaren Teil 22. Auf der Basis 20 ist ein passiver Testtransducer 200 angeordnet, während auf dem verformbaren Teil 22 ein aktiver Testtransducer 220 angeordnet ist.

Analog dazu weist der Sensor 1 auch einen Referenzcantilever 3 auf, der wiederum eine Basis 30 mit einem passiven Referenztransducer 300 aufweist, sowie einen verformbaren Teil 32 der einen aktiven Referenztransducer 320 aufweist.

Die Transducer 200, 220, 300, 320 sind jeweils über Elektroden 40 mit einer Elektronik 4 verbunden, die dazu in der Lage ist ein Messsignal der Transducer 200, 220, 300, 320 aufzuzeichnen oder weiterzuleiten, während die Elektronik 4 ebenfalls in der Lage ist die Transducer 200, 220, 300, 320 mit Strom und/oder Spannung zu versorgen.

Der Sensor 1 hat die Aufgabe, das Vorkommen und/oder die Konzentration und/oder die Menge eines Analyten 90 in einer Probe 9 anzuzeigen.

In der Figur 1 ist die Probe 9 eine Flüssigkeit, die beispielsweise durch Behandlung eines Abstriches, insbesondere eines Nasenabstriches oder eines Rachenabstriches eine Versuchsperson hergestellt wurde. Es kann aber auch sein, dass die Probe 9 Speichel oder Blut oder eine andere Körperflüssigkeit ist. Es kann aber auch sein, dass die Probe 9 eine Gurgelflüssigkeit ist, mit der die Versuchsperson gegurgelt hat. Es kann auch sein, dass die Probe 9 aus einer Gewebeentnahme oder aus einem anderen entnommenen Stoffes der Versuchsperson gewonnen und/oder synthetisiert wurde. Der Analyt 90 kann hierbei in der Probe gelöst sein, oder in einer ungelösten Art und Weise als Suspension oder Dispersion oder Emulsion vorliegen.

In jedem Fall soll mit dem Sensor 1 die Probe 9 mit Hinblick auf das Vorkommen und/oder eine Konzentration und/oder eine Menge eines Analyten 90 in der Probe 9 untersucht werden. Zu diesem Zweck ist auf den Testcantilever 2 eine Rezeptorschicht 24 aufgebracht, mit der ein Analyt 90 in Wechselwirkung treten kann, beziehungsweise eine Rezeptorschicht 24 die den Analyten 90 adsorbieren oder absorbieren kann. Bei der Adsorption würde der Analyt 90 an der Oberfläche der Rezeptorschicht 24 anhaften, während bei der Absorption der Analyt 90 in das Innere der Referenzschichtung 90 vordringen würde.

Sofern die Probe 9 einen Analyten 90 aufweist, kann dieser also mit der Rezeptorschicht 24 in Wechselwirkung treten. Dies kann dazu führen, dass sich die Oberflächenspannung des mit der Rezeptorschicht 24 belegten Abschnitts des verformbaren Teils 22 des Testcantilevers 2 ändert. Diese Änderung in der Oberflächenspannung kann durch den aktiven Testtransducer 220 registriert werden, was in der Elektronik 4 wiederum als Messsignal interpretiert wird.

Die Änderung in der Oberflächenspannung des mit der Rezeptorschicht 24 belegten Abschnitts des verformbaren Teils 22 des Testcantilevers 2 kann auch zu einer Verformung des verformbaren Teils 22 des Testcantilevers 2 führen. Der aktive Testtransducer 220 kann daher auch eine Verformung des verformbaren Teils des Testcantilevers 2 registrieren, was in der Elektronik 4 wiederum als Messsignal interpretiert wird.

Jedoch kann es bereits aufgrund der Wechselwirkung mit der Probenflüssigkeit 9 zur Registrierung einer Krafteinwirkung durch den aktiven Testtransducer 220 kommen, beispielsweise in dem lediglich die eine Oberflächenspannung der Flüssigkeit auf den verformbaren Teil 22 des Testcantilevers 2 wirkt. Für eine entsprechend resultierende Verformung oder Veränderung der Oberflächenspannung ist demnach in diesem Fall nicht das Vorhandensein eines Analyten 90 verantwortlich.

Um die Größe dieser Grundeinwirkung der Probe 9 auf den Testcantilever 2 festzustellen, wird gleichzeitig mit dem Testcantilever 2 auch der Referenzcantilever 3 mit der Probe 9 in Kontakt gebracht. Zu diesem Zweck weist der Referenzcantilever 3 eine Referenzschicht 34 auf, mit der ein Analyt 90 nicht in Wechselwirkung treten kann beziehungsweise eine Referenzschicht 24 die den Analyten 90 nicht adsorbieren oder absorbieren kann. Hierbei soll eine Wechselwirkung mit dem Analyten 90 vermieden werden, um eine Differenzierung zum Messsignal des Testcantilevers 2 zu ermöglichen.

Indem sowohl der Testcantilever 2 als auch der Referenzcantilever 3 mit der Probe 9 in Wechselwirkung treten, wechselwirken beider Cantilever 2, 3 in ähnlicher Art und Weise mit der Probe 9. Hierbei ist jedoch der Unterschied, dass der Testcantilever 2 über seine Referenzschicht 24 zusätzlich mit einem eventuell vorhandenen Analyten 90 in Wechselwirkung treten kann. Dementsprechend unterscheiden sich die Messsignale der aktiven Transducer 220, 320, sofern ein Analyt 90 in der Probe 9 vorkommt. Über die Größe des Unterschieds der Messsignale kann demnach im einfachsten Fall auf die Menge des Vorkommens des Analyten 90 in der Probe 9 geschlossen werden.

Der Testcantilever 2 und der Referenzcantilever 3 vermessen das Vorkommen des Analyten 19 der Probe 9 jedoch an unterschiedlichen Positionen. An unterschiedlichen Positionen der Probe kann es zu unterschiedlichen Umgebungsbedingungen kommen, wie beispielsweise Temperaturschwankungen oder Konzentrationsgradienten etc. Diese unterschiedlichen Umgebungsbedingungen können mit den passiven Transducer 200, 300 vermessen werden.

Die passiven Transducer 200, 300 sind auf der Basis angeordnet und detektieren bevorzugt bei einer Verformung oder Änderung in der Oberflächenspannung des verformbaren Teils 22, 32 der Referenz- beziehungsweise Testcantilever 2, 3 kein Messsignal. Jedoch kann der Grundpegel des Messsignals der passiven Transducer 200, 300 aufgrund dieser unterschiedlichen Umgebungsbedingungen beeinflusst werden. Indem für jeden Messwert der aktiven Transducer 220, 23 über die passiven Transducer 200, 301 jeweils ein Vergleichswert bereitgestellt wird, der die Umgebungsbedingungen isoliert betrachtet, kann der Einfluss der Umgebungsbedingungen auf die Messsignale der aktiven Transducer 220, 320 bestimmt und reduziert beziehungsweise herausgerechnet oder isoliert werden.

Demnach kann über den Sensor 1 das Vorkommen eines Analyten 90 in einer Probe 9 isoliert analysiert werden, indem durch eine Vielzahl an Messpunkten auf den Referenz- und Testcantilever 3, 2 der Einfluss von Wechselwirkungen, die nicht dem Analyten 90 zuzuordnen sind, reduziert und isoliert werden. Dies ermöglicht eine hohe Messgenauigkeit des Vorkommens des Analyten 90 in der Probe 9.

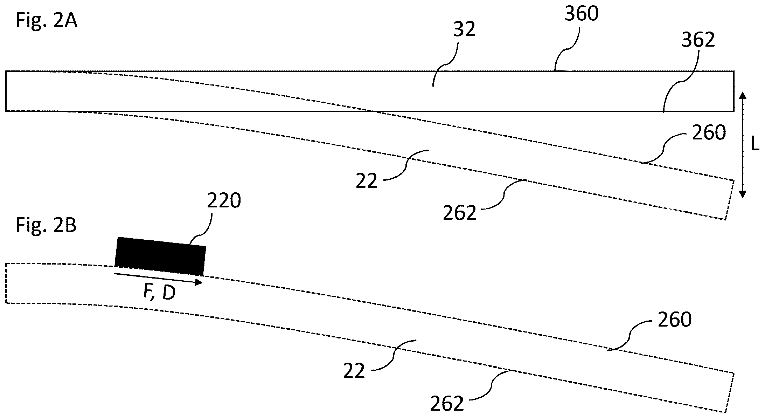

In Figur 2A ist der Vergleich der verformbaren Teile 32, 22 der Referenz-und Testcantilever 3, 2 bei einer Verformung und Längsdehnung gezeigt. Der verformbare Teil 32 des Referenzcantilevers 3 weist eine obere Oberfläche 360 und eine untere Oberfläche 362 auf. Ebenso weist der verformbare Teil 22 des Testcantilevers 2 eine obere Oberfläche 262 und eine untere Oberfläche 262 auf. Sofern ein Analyt 90 der Probe 9 mit dem Testcantilever 2, beziehungsweise mit der Rezeptorschicht 24 in Wechselwirkung tritt, findet eine Änderung der Oberflächenspannung und damit beispielsweise eine Verformung des verformbaren Teils 22 vom ortsfesten Teil (der in die Basis des Testcantilevers übergeht) hin zum frei beweglichen Teil des verformbaren Teils 22 statt. Die gezeigte Auslenkung L ist hierbei gegeben durch die relative Auslenkung zwischen dem verformbaren Teil 32 des Referenzcantilevers 3 und dem verformbaren Teil 22 des Testcantilevers 2 aufgrund der Wechselwirkung mit dem Analyten 90.

Die Verformung des verformbaren Teils 22 des Testcantilevers 2 ist in Figur 2B gezeigt. Ursächlich hierfür ist, dass sich die obere Oberfläche 260 und die untere Oberfläche 262 unterschiedlich stark dehnen. Aufgrund der großen Dehnung D an der oberen Oberfläche 260, kann ein darauf aufgebrachter aktiver Transducer 220 eine Änderung der Oberflächenspannung und/oder eine Dehnungskraft F registrieren. Die registrierte Änderung der Oberflächenspannung und/oder der Dehnungskraft F kann hierbei durch dem aktiven Transducer 220 in ein elektronisches Signal umgewandelt werden beziehungsweise ein vorhandenes elektronisches Signal, beispielsweise eine anliegende Spannung, beeinflussen. Dies kann beispielsweise dadurch geschehen, dass der Transducer den Widerstand ändert, sofern er eine Dehnungskraft F erfährt, die wiederum in einer Dehnung des Transducers 220 resultiert.

Der Transducer könnte auch eine Stauchung der Oberfläche, auf der er angeordnet ist, detektieren. In den gezeigten Ausführungsformen sind die Transducer aber immer an Oberflächen angeordnet, bei denen eine Dehnung erwartet wird.

Die Dehnung und/oder Veränderung der Oberflächenspannung und/oder Kraft, die der Transducer detektiert, kann jedoch auch eine Biegungskraft oder eine Scherkraft sein oder durch eine Biegungskraft oder Scherkraft hervorgerufen sein oder allgemein auf dem Elastizitätsmodul des jeweiligen Cantilevers beruhen. Insbesondere ergibt sich durch die Befestigung des verformbaren Teils 22, 32 an der Basis 20, 30, dass der verformbare Teil 22, 32 sich aufgrund einer Krafteinwirkung, die durch eine Veränderung der Oberflächenspannung des Testcantilevers entlang einer Biegekurve ausrichtet. Die resultierende Biegekurve ist insbesondere gegeben durch die Geometrie, insbesondere das Flächenträgheitsmoment des Cantilevers, sowie durch die Masse des Cantilevers und das Elastizitätsmodul. Die Biegekurven kann beispielsweise gemäß der Balkentheorie beschrieben werden.

Durch die sich auf der Unterseite und der Oberseite des Cantilevers unterscheidenden Oberflächenspannungen kommt es entsprechend zu der beschriebenen Verformung oder Dehnung des Cantilevers.

Über die Balkentheorie ist es beispielsweise möglich vorherzusagen, an welcher Stelle des verformbaren Teils 22, 32 die Dehnung D am größten ist. Es ist möglich, dass an dieser Stelle der aktive Transducer 220, 320 angeordnet wird, um ein optimales Signalrauschverhältnis zu erzielen und um möglichst sensitiv auf die Dehnungen zu reagieren. Bei der genauen Positionierung der Transducer sollten jedoch auch andere Rahmenbedingungen berücksichtigt werden.

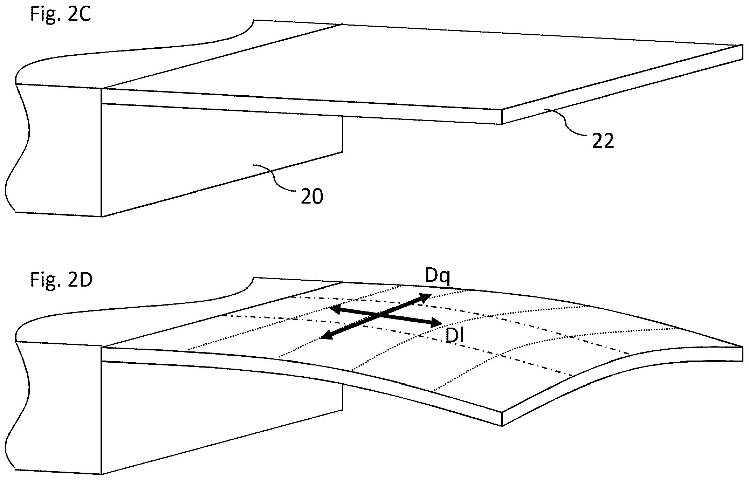

Insbesondere spielt die Ausrichtung der Transducer relativ zur Ausrichtung der Cantilever eine wichtige Rolle. In Figur 2C ist beispielsweise ein unausgelenkter Cantilever gezeigt. Kommt der Cantilever in Kontakt mit dem Analyten, so ändert sich die Oberflächenspannung und es kommt zu einer Verformung des Materials, wie in Figur 2D gezeigt. In Figur 2D ist dargestellt, dass der Cantilever eine Verformung senkrecht zur Basis 20, beziehungsweise zur Biegekante erfährt. Dies geht mit einer Längsausdehnung DI der oberen Oberfläche einher. Gleichzeitig findet eine Verformung parallel zur Basis 20, beziehungswiese zur Biegekante statt, die mit einer Querausdehnung Dq der oberen Oberfläche einhergeht. Durch die Geometrie des Cantilevers kann festgelegt werden, entlang welcher Richtung eine größere Dehnung D bewirkt wird. Insbesondere kann der Transducer entlang dieser Richtung ausgerichtet werden, um ein besonders großes Messsignal zu erzeugen.

Durch eine Überhöhung einer mechanischen Dehnung am Ort des Transducers kann das vom Transducer ermittelte Signal noch weiter verbessert werden. Eine solche Überhöhung kann beispielsweise durch die Anordnung und Form der Elektroden erreicht werden.

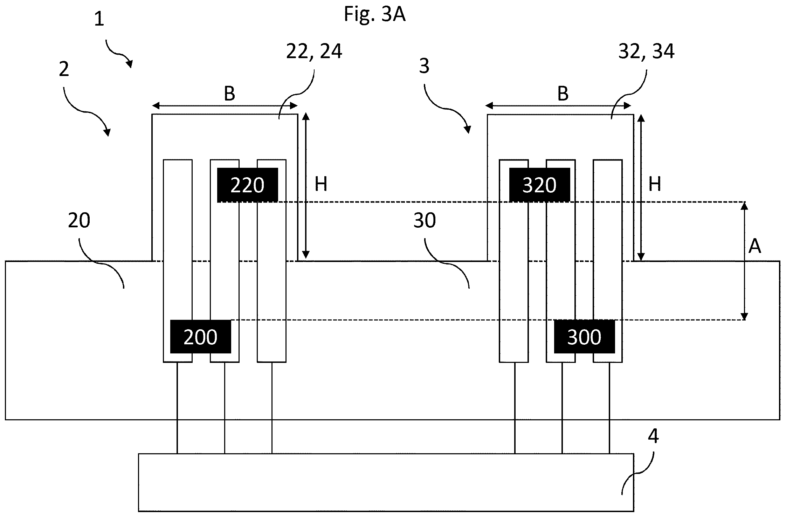

In Figur 3A ist eine weitere Ausführungsformen des Sensors 1 gezeigt. Insbesondere weisen der Referenzcantilever 3 und der Testcantilever 2 identische geometrische Abmessungen auf, insbesondere entsprechen die Höhe, Breite und Dicke des Referenzcantilevers 3 der Höhe, Breite und Dicke des Testcantilevers 2. Dadurch wird eine Dehnung D auf den oberen Oberflächen 260, 360 erzeugt. In dem die geometrischen Abmessungen der Cantilever 2, 3 identisch sind, wird dementsprechend auch eine gleiche Abhängigkeit des Messsignals von der Dehnung erwartet.

Bevorzugt ist die Breite B der Cantilever gleich der Höhe H der Cantilever 2, 3, dadurch wird eine besonders große Dehnung D an der oberen Oberfläche 260, 360 des Cantilevers 2, 3 ermöglicht. Beispielsweise sind dabei die Cantilever weniger als 100 μm breit, weniger als 100 μm lang und weniger als 1 μm dick, insbesondere 50 μm breit, 50 μm lang und 0,3 μm dick.

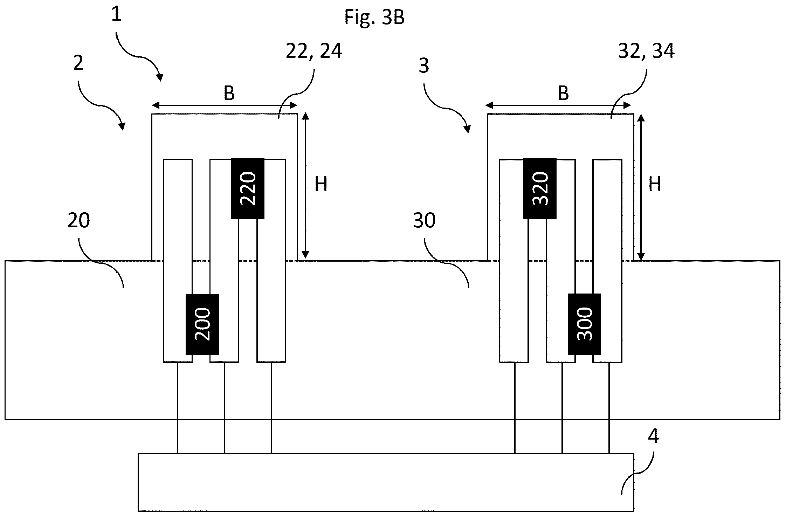

In der Ausführungsformen des Sensors 1 in Figur 3 sind die Basen 30, 20 des Referenz- und Testcantilevers 3, 2 zudem auf derselben Gesamtbasis angeordnet. Dementsprechend gibt es eine direkte mechanische Verbindung und Wechselwirkung der Cantilever über die Gesamtbasis. Dadurch können beispielsweise die unterschiedlichen Umgebungseinflüsse auf die Cantilever 22, 3 reduziert werden, da die Cantilever 2, 3 näher aneinander angeordnet werden können. Insbesondere können die Basen 30, 20 der Referenz-und Testcantilever 3, 2 auch einteilig ausgebildet sein. Dadurch wird sichergestellt, dass auch die Basen gleiche materialspezifische Bindungseigenschaften aufweisen, sodass die Messergebnisse der passiven und aktiven Transducer 200, 220, 300, 320 gut mit einander vergleichbar werden.

Der Abstand A der aktiven Transducer 320, 220 von den passiven Transducer 300, 200 wird entlang der Höhenrichtung H der Cantilever gemessen. Der Abstand A ist insbesondere kleiner als 100 μm, wodurch sichergestellt wird, dass die Transducer möglichst nahe einander angeordnet werden, sodass beispielsweise räumliche Umgebungseinflüsse auf die Transducer reduziert werden.

In Figur 3B ist eine weitere Ausführungsform gezeigt, bei der die Transducer 200, 220, 300 und 320 senkrecht zur Basis 20, 30 ausgerichtet sind. Während mit der Querausrichtung der Transducer entlang der Biegekante in Figur 3A noch eine Querdehnung der Cantilever 22, 32 gemessen wird, wird in Figur 3B eine Längsdehnung der Cantilever 22, 32 gemessen.

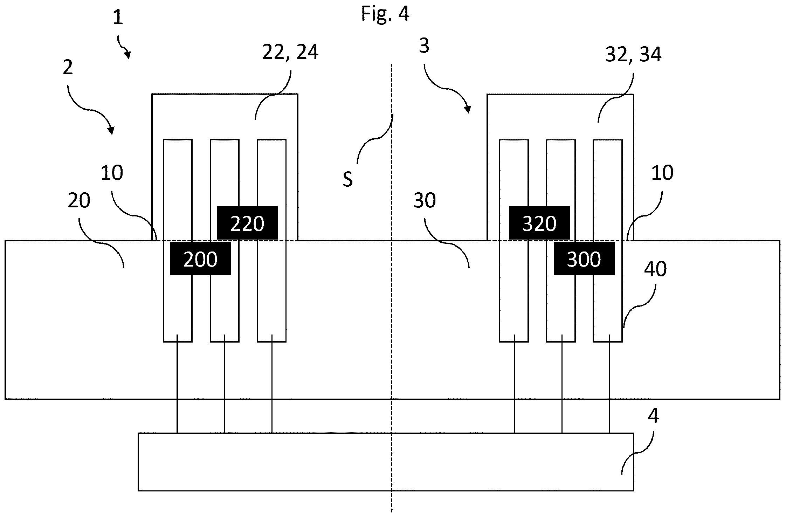

In Figur 4 ist diesbezüglich eine bevorzugte Ausführungsform gezeigt, bei der die aktiven Transducer 320, 220 und die passiven Transducer 300, 200 jeweils an der Biegekante 10 der Cantilever 3, 2 anliegen. In dem alle Transducer 320, 300, 220, 200 an der Biegekante 10 anliegen, ist der geringstmögliche Abstand Ader Transducer realisiert 320, 300, 220, 200. Des Weiteren sind dieser Ausführungsform die Elektroden 40 als auch die Transducer 320, 300, 220, 200 spiegelsymmetrisch zu einer Spiegelsymmetrieachse S orientiert. Insbesondere sind die Transducer 320, 300, 220, 200 somit spiegelsymmetrisch zueinander orientiert.

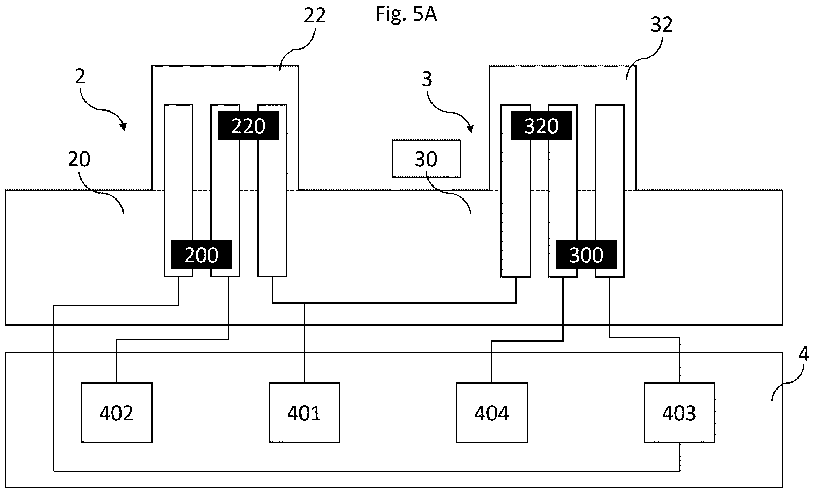

In Figur 5A ist eine weitere Ausführungsform des Sensors 1 gezeigt. Die Transducer 300, 320, 200, 220 sind über die Elektroden 401, 402, 403, 404 kontaktiert. Insbesondere ist der aktive Transducer 220 mit dem aktiven Transducer 320 über die Elektrode 401 verbunden. Des Weiteren ist der passive Transducer 200 mit dem passiven Transducer 300 über die Elektrode 403 verbunden. Der aktive Transducer 220 ist zudem mit dem passiven Transducer 200 über die Elektrode 402 verbunden, wohingegen der aktive Transducer 320 mit dem passiven Transducer 300 über die Elektrode 404 verbunden ist. Somit ergeben sich insgesamt vier Elektroden über die die Transducer miteinander elektrisch kontaktiert werden. Eine elektrische Kontaktierung kann hierbei insbesondere erreicht werden, indem die Transducer auf die Elektroden aufgebracht werden, sodass eine leitfähige Verbindung entsteht. Da die Transducer eine Dicke aufweisen kann es insbesondere sein, dass bei einem nachträglichen Aufbringen von Elektroden, an den Kanten der Transducer keine leitfähige Kontaktierung zu den Elektroden erzielt werden würde. Dies ist erst sichergestellt, wenn die Dicke der Elektroden größer als die Dicke der Transducer ist.

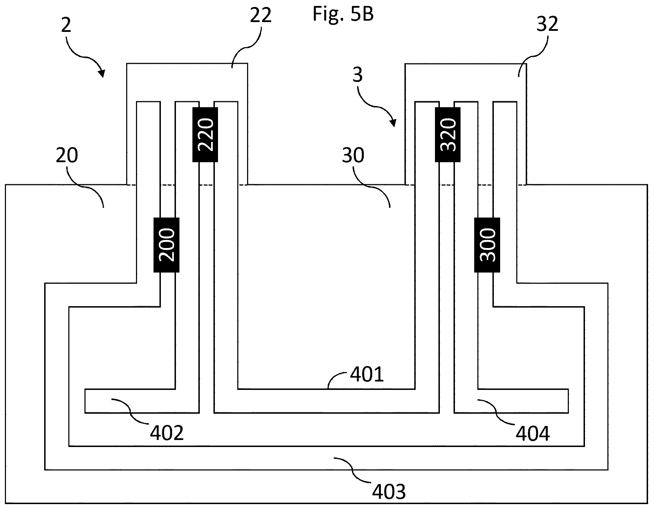

In Figur 5B ist eine weitere Ausführungsform des Sensors 1 gezeigt. Die Elektroden, die die Transducer 200, 220, 300, 320 kontaktieren sind insgesamt spiegelsymmetrisch aufgebaut. Durch die Elektroden laufen Ströme beziehungsweise es liegen Spannungen an, sodass bei einer asymmetrischen ausbilden dieser Elektroden zu einem asymmetrischen übersprechen elektrischen Signale auf die anderen Elektroden kommen kann. Durch diese wechselseitige Beeinflussung kann es zur Erzeugung eines Steuersignals zwischen den Elektroden kommen, was jedoch durch die symmetrische Bauweise vermieden werden kann.

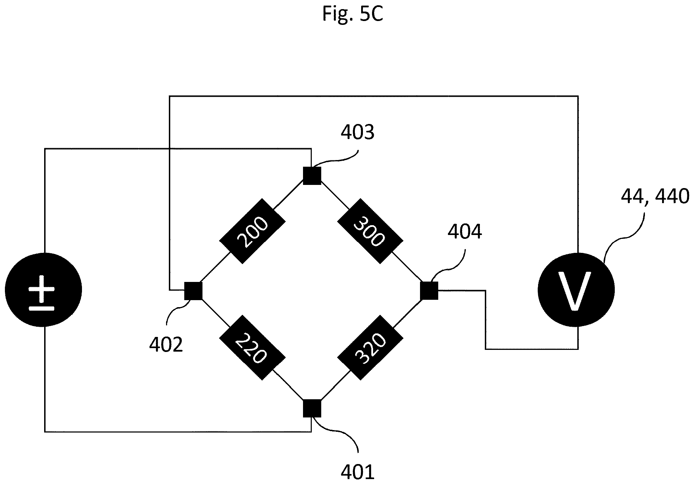

Die Transducer 200, 220, 300, 320 sind insbesondere in einer sogenannten Vollbrücke elektrisch verschaltet. Die Schaltung der Vollbrücke ist in Figur 5C gezeigt. Bei der Vollbrücke wird eine Gleichspannung oder Wechselspannung zwischen den Elektroden 403, 401 angelegt. Zwischen diesen Elektroden wirken die passiven und aktiven Transducer als Spannungsteiler, aufgrund ihrer elektrischen Widerstände. Eine Vollbrücke in der gezeigten Form hat den Vorteil, dass zwischen den Elektroden 402, 404 keine Spannung aufgebaut wird, sofern das Verhältnis der Widerstände des passiven Transducers 200 zum aktiven Transducer 220 des Testcantilevers 2 gleich dem Verhältnis der Widerstände des passiven Transducers 300 zum aktiven Transducer 320 des Referenzcantilevers 3 ist. Es genügt also insbesondere die Abweichung eines Widerstandes, um die Widerstandsverhältnisse zu ändern, und um so eine Spannung zwischen den Elektroden 402, 404 aufzubauen.

Wenn der Referenzcantilever 3 und der Testcantilever 2 mit der Probe 9 und dem Analyten 90 in Wechselwirkung treten, so erfahren beide verformbaren Teile 22, 32 beispielsweise eine Änderung der Oberflächenspannung, die für den verformbaren Teil 22 des Testcantilevers 2 größer ist als für den verformbaren Teil 32 des Referenzcantilevers 3. Demzufolge wird der Widerstand des aktiven Testtransducers 220 des verformbaren Teils 22 des Testcantilevers 2 in stärkerem Maße variieren als für den aktiven Referenztransducer 320 des verformbaren Teils 32 des Referenzcantilevers 3. Sofern sich die Widerstände der passiven Transducer 200, 300 nicht ändern oder zumindest gleich ändern, ergibt sich eine Änderung der Widerstandsverhältnisse aus der Verformung des verformbaren Teils 22 des Testcantilevers 2 aufgrund der Wechselwirkung mit dem Analyten 90 der Probe 9, die spezifisch mit der Referenzschicht 24 des Testcantilevers 2 wechselwirkt. Bei einer solchen Wechselwirkung wird dementsprechend eine Spannung zwischen den Elektroden 402, 404 aufgebaut, so dass eine Krafteinwirkung auf den aktiven Testtransducer 220 relativ zum aktiven Referenztransducer 320 als Brückenquerspannung VB angezeigt werden kann. Bevorzugt skaliert die Brückenquerspannung VB mit dem Vorkommen des Analyten 90 in der Probe 9, sodass eine quantitative Auswertung des Messsignals ermöglicht wird.

Ein Brückenquerspannungsdetektor 44 kann die Brückenquerspannung VB nach außen anzeigen oder weiterleiten, sodass für den Anwender des Sensors 1 sichtbar wird, dass eine Brückenquerspannung VB anliegt. Insbesondere kann ein solcher Brückenquerspannungsdetektor 44 auch durch einen AD-Wandler gegeben sein wobei der AD-Wandler die Brückenquerspannung VB in ein Digitalsignal umwandelt, welches zur externen Messvorrichtung weitergeleitet werden kann. Insbesondere kann der AD-Wandler in zwei verschiedenen Messmodi betrieben werden. Der erste Messmodus ist der differentielle Messmodus bei dem die Brückenquerspannung VB gemessen wird und somit ein relativer Messwert für die Verformung der beiden Referenz-und Testcantilever 3, 2 erzeugt wird. In diesem differentiellen Messmodus wird gewissermaßen das Messsignale aller Transducer 200, 22, 300, 23 berücksichtigt, sodass das Ausgabesignal des AD-Wandlers ein von Umgebungseinflüssen bereinigtes Messsignale ist, durch welches sich auf die relative Verformung der verformbaren Teile 32, 22 und somit auf das Vorkommen eines Analyten 90 schließen lässt.

Der zweite Messmodus ist der sogenannte absolute Messmodus. In dem absoluten Messmodus wird nicht die Brückenquerspannung detektiert, sondern vielmehr die Signale an den Elektroden 402 beziehungsweise 404 isoliert voneinander abgegriffen, sodass eine Aussage über die jeweiligen Auslenkungen der verformbaren Teile 32, 22 getroffen werden kann. Diese Information bleibt dem Benutzer im differentiellen Messmodus verwehrt.

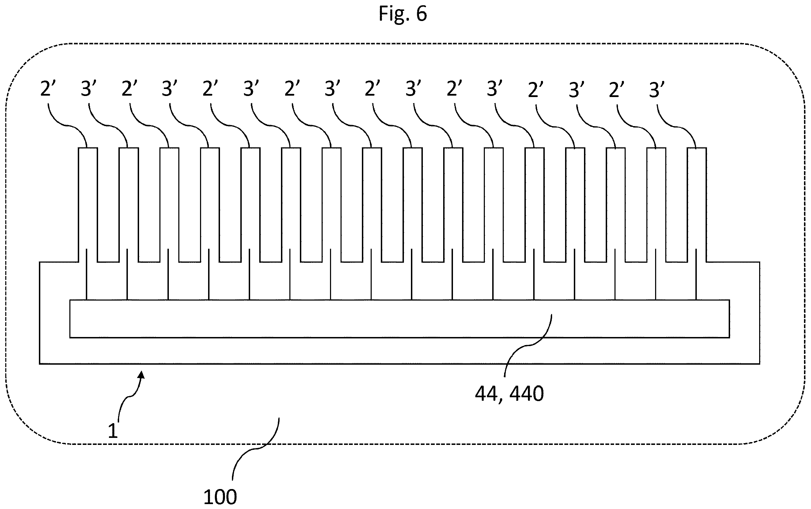

In Figur 6 ist eine weitere Ausführungsformen des Sensors 1 gezeigt. Der Sensor 1 umfasst hierbei mehrere Cantileverpaare, wobei hier jedes Cantileverpaar einen Referenzcantilever 3' und einen Testcantilever 2' umfasst. Die Referenzcantilever 3' und Testcantilever 2', beziehungsweise die entsprechenden Transducer, sind wie in Figuren 5A bis C über eine Elektrodenschaltung miteinander in elektrischer Verbindung, sodass für jedes Cantileverpaar eine Brückenquerspannung VB' abgegriffen werden kann. Die Brückenquerspannung VB' kann von jedem Cantileverpaar vom AD-Wandler 440, beziehungsweise vom Brückenquerspannungsdetektor 44 abgegriffen werden. Insbesondere kann im AD-Wandler 440 über eine AD-Wandlerlogik beispielsweise das Messsignal eines bestimmten Cantileverpaars ausgegeben werden oder das integrierte Messsignale aller Cantileverpaare ausgegeben werden oder eine Kombination davon. Somit ist es insbesondere möglich über verschiedene Cantileverpaare die Messsignale zu Mitteln, sodass das Anzeigen eines Vorkommens eines Analyten 90 mit höherer statistischer Signifikanz geschieht. Es ist aber auch möglich, dass auf den verschiedenen Cantileverpaaren verschiedene Referenz- und Rezeptorschichten 34, 24 aufgebracht sind, sodass mit einem solchen Sensor 1 die Probe 9 auf verschiedene Analyten 90 gleichzeitig untersucht werden kann. Es ist aber beispielsweise auch möglich, dass ein einziger Referenzcantilever 3 als Referenz für mehrere Testcantilever 2 dient.

Insbesondere ist der Sensor 1 mit der Vielzahl an Cantileverpaaren auf einem Chip 100 ausgebildet. Ein Chip kann hierbei bedeuten, dass der Sensor 1 aus einem einzigen Substrat angefertigt wurde, sodass beispielsweise die verschiedenen Cantilever 2, 3 in mechanischer Verbindung miteinander stehen. Es kann aber auch sein das der Chip 100 eine weitere elektronische Schaltung umfasst, die beispielsweise eine CMOS Schaltung ist, also eine Halbleiterschaltung ist, die die Brückenquerspannung VB' abgreift und direkt weiterverarbeitet. Eine solche Halbleiterschaltung in Kombination mit einem Sensor wird auch System-On-A-Chip genannt.

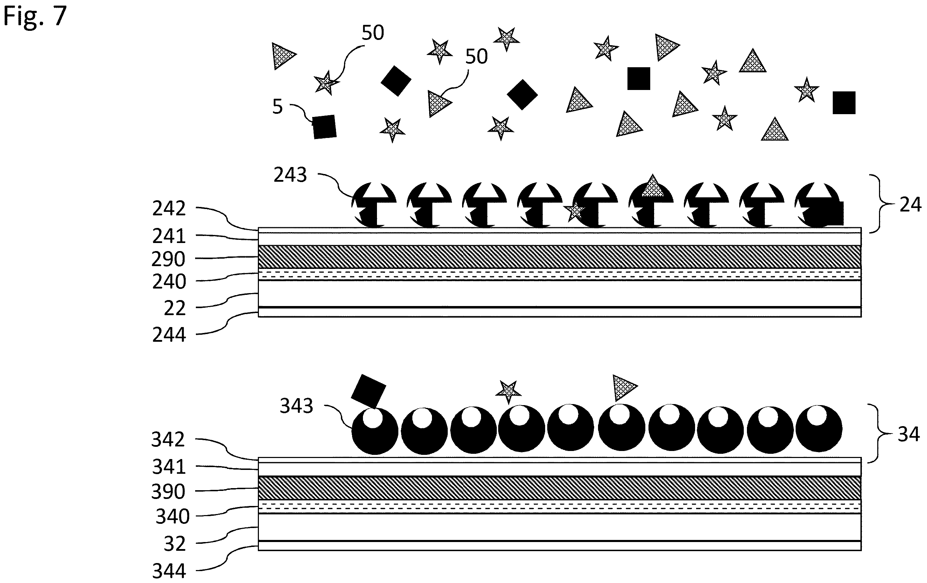

In Figur 7 ist schematisch der Aufbau der verschiedenen verformbaren Teile 22, 32 der Referenzbeziehungsweise Testcantilever 3, 2 gezeigt. Der Aufbau der Cantilever ist bis auf die Rezeptorschicht beziehungsweise die Referenzschicht identisch, so dass eine Wechselwirkung mit der Probe beziehungsweise dem umgebenden Medium als auch die mechanische Ausgestaltung des Cantilevers weitestgehend gleich ist.

Auf dem verformbaren Teil 32, 22 des Referenzbeziehungsweise Testcantilevers 3, 2 ist eine Referenz-Wandlerschicht 340 beziehungsweise eine Test-Wandlerschicht 240 aufgebracht, die einerseits Elektroden für Referenz- und Testtransducer (in Figur 7 nicht gezeigt) ausbildet und andererseits optional eine Haftvermittlung zwischen der Oberfläche des verformbaren Teils 32, 22 und einer Referenz-Funktionsschicht 390 beziehungsweise Test-Funktionsschicht 290 zu realisieren.

Auf der Oberfläche der Referenz-Funktionsschicht 390 beziehungsweise Test-Funktionsschicht 290 kann eine Monoschicht 341, 241 aufgebracht sein, auf der eine Rezeptorschicht 242 oder eine Referenzschicht 342 aufgebracht ist.

Die die Funktionsschicht 390, 290 hat die Aufgabe, einen asymmetrischen Schichtaufbau des Cantilevers 3, 2 hervorzurufen, so dass es einen möglichst großen Unterschied in der Ausdehnung der oberen Oberfläche des Cantilevers und der unteren Oberfläche des Cantilevers gibt. Die Wandlerschicht 340, 240 kann insbesondere Gold umfassen oder aus Gold bestehen, wobei die Transducer entsprechend über die in der Wandlerschicht 340, 240 angeordneten Elektroden kontaktiert sind.

Auf die Wandlerschicht 340, 240, insbesondere die Gold aufweisende oder aus Gold bestehende Wandlerschicht 340, 240, kann mittels der Funktionsschicht 390, 290 auch eine elektrische Isolierung aufgebracht werden, um gegenüber der Umgebung eine Isolierung bereit zu stellen.

Die Funktionsschicht 390, 290 kann auch zum Ausgleich der durch die Strukturierung der Elektroden der Wandlerschicht 340, 240 und/oder der Ausbildung der Transducer hervorgerufenen Oberflächenunebenheiten oder Gräben dienen.

Auf der Funktionsschicht 390, 290 kann eine sogenannte selbstorganisierende Monoschicht 341, 241 aufgebracht werden, welche die verbleibenden Oberflächenunebenheiten der darunterliegenden Funktionsschicht 390, 290 ausgleichen kann und gleichzeitig für eine Haftvermittlung für weitere Schichten, nämlich die Referenz- beziehungsweise Rezeptorschichten 34, 24, bereitstellt.

Der Aufbau der Referenz- beziehungsweise Rezeptorschicht 34, 24 kann unterschiedlich sein und an die jeweiligen Anforderungen der Analyse angepasst sein.

In einer bevorzugten Ausführung basieren beide Schichten jedoch auf einer Schicht, die das sogenannte Protein A 242 umfassen kann, welches einerseits an die selbstorganisierende Monoschicht 241, 341 bindet, jedoch auf seiner Oberfläche auch Antikörper 243 beziehungsweise Isotypkontroll-Antikörper 343 aufweisen und binden kann.

Die Antikörper 243 sind Proteine die auf ein Antigen 5 reagieren, beziehungsweise mit diesem binden und somit beispielsweise im menschlichen Immunsystem Viruszellen markieren, sodass das Immunsystem den markierten Virus entsprechend vernichten kann, um beispielsweise einen Virusausbruch einzudämmen oder zu verhindern. Die Antikörper 243 sind weitestgehend spezifisch auf das Antigen 5, können jedoch auch mit anderen ähnlichen Antigenen 50 in Wechselwirkung treten. In der Figur 7 ist gezeigt, dass der Antikörper 243 gewissermaßen mit dem Antigen 5 und den ähnlichen Antigenen 50 in Wechselwirkung treten kann.

Im Gegensatz zum Antikörper 243 ist der Isotypkontroll-Antikörper 343 ein Protein welches bevorzugt ultrahochspezifisch nicht mit dem Antigen 5 in Wechselwirkung tritt. Dadurch kann eine Wechselwirkung mit einem spezifischen Antigen 5 quasi ausgeschlossen werden. Dies ist in der Figur 7 dadurch gezeigt, dass das Isotypkontroll-Antikörper 343 nur mit zwei ähnlichen Antigenen 50 in Wechselwirkung treten kann, jedoch nicht mit dem hier schematisch quadratisch dargestellten.

Indem der Testcantilever 2 einen Antikörper 243 aufweist und der Referenzcantilever 3 einen Isotypkontroll-Antikörper 343 aufweist wird sichergestellt, dass in der Probe 9 der Analyt 90, sofern der Analyt 90 ein Antigen 5 ist, hier nur mit dem Testcantilever 2 in Wechselwirkung treten kann. Dadurch ist sichergestellt, dass die von dem Analyten hervorgerufene relative Verformung des Testcantilevers 2 im Vergleich zur Verformung des Referenzcantilevers 3 nur auf der Anwesenheit des Analyten 90, beziehungsweise des Antigen 5 basiert. Demnach ist mit diesem Sensor 1 möglich ein Antigen 5 sicher und schnell zu detektieren.

Im Gegensatz zur oberen Oberfläche der Cantilever ist die untere Oberfläche der Cantilever passiviert. Eine solche Passivierung kann dazu führen, dass eine Wechselwirkung, beziehungsweise Bindung, beziehungsweise Absorption oder Absorption eines Analyten 90 der Probe 9 in oder auf den Cantilever vermieden wird. Insbesondere trägt eine solche Passivierungsschicht jedoch auch dazu bei, die Asymmetrie des Schichtaufbaus zu vergrößern, um einen möglichst großen Dehnungseffekt an der oberen Oberfläche des Cantilevers 3, 2 hervorzurufen. Insbesondere kann die Passivierungsschicht Trimethoxisilan und/oder eine Blocking-Substanz umfassen.

Der gezeigte Sensor kann insbesondere dazu verwendet werden die Antigene 5 eines Sars-CoV2 Virus oder eines anderen Virus zu detektieren. Hierzu umfasst die Rezeptorschicht 24 des Testcantilevers 2 beispielsweise Sars-CoV2-Antikörper, während die Referenzschicht 34 Sars-CoV2-spezifische Isotypkontroll-Antikörper umfasst. Dementsprechend wird von dem Sensor 1 ein Messsignal erzeugt, wenn in der Probe 9 die Antigene 5 eines Sars-CoV2 Virus vorhanden sind und diese sich an dem Testcantilever 2, beziehungsweise der Rezeptorschicht 24 anlagern.

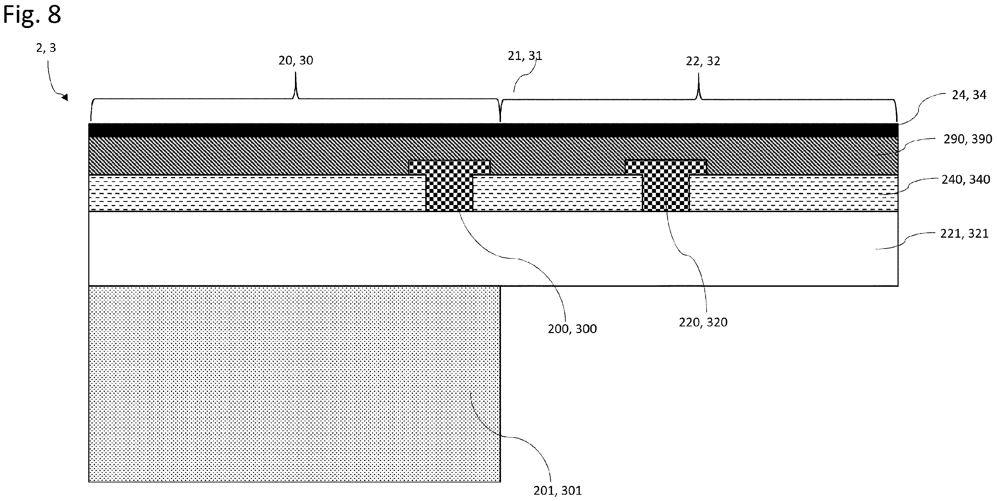

In Figur 8 ist schematisch ein Referenzcantilever 3 bzw. Testcantilever 2 gezeigt. Der Cantilever 3, 2 ist unterteilt in eine Basis 30, 20 und einen verformbaren Teil 32, 22, wobei die Grenze zwischen der Basis 30, 20 und dem verformbaren Teil 32, 22 an der Biegekante 31, 21 liegt.

In der dargestellten Ausführungsform umfasst der Referenzcantilever 3 bzw. Testcantilever 2 mehrere Schichten oder ist daraus aufgebaut. Hierbei umfasst der Cantilever 3, 2 einen Grundkörper 301, 201, der beispielsweise Silizium umfasst oder daraus gefertigt ist, auf dem eine erste Trägerschicht 321, 221, beispielsweise bestehend aus SiN, aufgebracht ist, welche über die Kante des Grundkörpers 301, 201 hinausragt und sich somit sowohl über Basis 30, 20 und den verformbaren Teil 32, 22 erstreckt und damit strukturell auch den verformbaren Teil 32, 22 des Cantilevers 3, 2 ausbildet.

Weiterhin umfasst der Cantilever 3, 2 eine Test-Wandlerschicht 240 bzw. Referenz-Wandlerschicht 340, welche als leitfähige Schicht ausgeführt ist und zur elektrischen Kontaktierung eines passiven Referenz- bzw.

Testtransducers 300, 200 auf der Basis 30, 20 und eines aktiven Referenz- bzw. Testtransducers 320, 220 auf dem verformbaren Teil 32, 22 ausgebildet ist. In der Test-Wandlerschicht 240 bzw. der Referenz-Wandlerschicht 340 können entsprechend strukturierte Leiterbahnen vorgesehen sein, die die vorgesehene Leiterstruktur zur Kontaktierung der jeweiligen Transducer ausbilden.

Die jeweiligen Transducer 300, 320, 200, 220 können in der jeweiligen Wandlerschicht 340, 240 aufgenommen sein. Mit anderen Worten kann die Struktur der Leiterbahnen der Wandlerschicht so ausgebildet sein, dass die Transducer 300, 320, 200, 220 direkt mit der ersten Trägerschicht 321, 221 in Kontakt stehen beziehungsweise auf dieser aufgebracht sind und die Leiterbahnen lateral daran anschließen.

Die Wandlerschicht 340, 240, die in gezeigter Ausführung als leitfähige Schicht ausgeführt ist, kann zum Zweck einer erhöhten Oberflächendehnung in der Wandlerschicht gleichzeitig als Aktivierungsschicht dienen, sodass die Sensitivität des aktiven Referenz- bzw. Testtransducers 320, 220 bei Verformung des Cantilevers erhöht ist.

Wie in der Figur durch die Lücken in der Wandlerschicht 340, 240 an den Stellen des aktiven und passiven Referenz- bzw. Testtransducers 300, 200 und 320, 220 angedeutet, ist die als leitfähige Schicht ausgeführte Wandlerschicht 340, 240 zur geeigneten elektrischen Kontaktierung der Referenz- bzw. Testtransducer 320,220, 300, 200 zu Elektroden strukturiert.

Der Cantilever 3, 2 umfasst weiterhin eine Test-Funktionsschicht bzw. Referenz-Funktionsschicht 390, 290, welche zwischen der Wandlerschicht 340, 240 und einer Rezeptorschicht bzw. Referenzschicht 34, 24 angeordnet ist. In der dargestellten Ausführungsform deckt die Funktionsschicht 390, 290 die komplette Fläche der Wandlerschicht 340, 240 ab, inklusive des aktiven und passiven Referenz- bzw. Testtransducers 320, 220 und 300, 200. Die Rezeptorschicht bzw. Referenzschicht 32, 22 ist auf der äußeren Oberfläche der Funktionsschicht 390, 290 angebracht, um einen unmittelbaren Kontakt mit einer zu untersuchenden Probe sicherzustellen.

Die Test-Funktionsschicht 290 bzw. Referenz-Funktionsschicht 390 kann dadurch beispielsweise die durch die Strukturierung der Elektroden in der Wandlerschicht 340, 240 hervorgerufenen Oberflächenunebenheiten ausgleichen oder zumindest mindern.

Die Test-Funktionsschicht 290 bzw. Referenz-Funktionsschicht 390 kann dadurch beispielsweise auch eine elektrische Isolierung der Elektroden in der Wandlerschicht 340, 240 sowie der Transducer 200, 220, 300, 320 gegenüber der Rezeptorschicht bzw. der Referenzschicht 32, 22 erreichen.

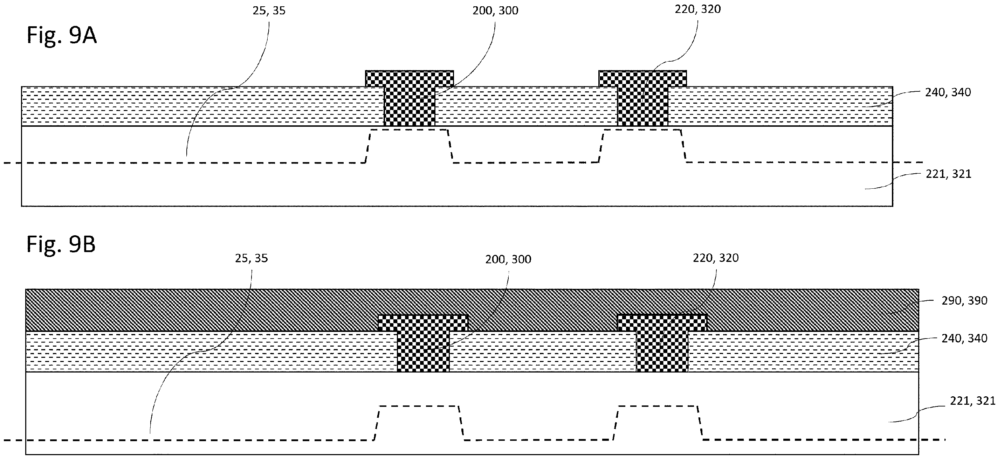

Figur 9A zeigt eine beispielhafte Schichtstruktur des Cantilevers, insbesondere umfassend eine Trägerschicht 321, 221 und eine Wandlerschicht 340, 240, sowie passive Transducer 300, 200 und aktive Transducer 320, 220.

Zusätzlich ist in Form einer gestrichelten Linie eine neutrale Achse 35, 25 des Cantilevers 3, 2 eingezeichnet.

Die neutrale Achse 35, 25 ist dadurch gekennzeichnet, dass bei Verbiegung des Cantilevers entlang besagter Achse keine Längenänderung stattfindet. Wie in der Figur beispielhaft dargestellt, ist der Verlauf der neutralen Achse signifikant durch die Schichtstruktur beeinflusst. Bei einem homogenen Material, insbesondere einem Material mit homogenem Elastizitätsmodul, verläuft die neutrale Achse entlang der geometrischen Mitte des Materials (betrachtet man eine Verbiegung/-Verformung des Materials quer zu dieser neutralen Achse).

Bei mehreren Schichten und gegebenenfalls unterschiedlichen Elastizitätsmodulen und Dicken der beteiligten Schichten kann die neutrale Achse auch abseits der geometrischen Mitte der Schichtstruktur liegen, so wie durch die entsprechende Gleichung im allgemeinen Beschreibungsteil verdeutlicht.

Bevorzugt sollte die neutrale Achse 35, 25 möglichst weit von den aktiven Transducern entfernt liegen, um die Sensitivität des Sensors zu maximieren. Mit anderen Worten liegt die neutrale Achse 35, 25 bevorzugt so, dass die Änderung der Oberflächenspannung beziehungsweise die Längenänderung an der Position der aktiven Transducer möglichst groß ist.

Die neutrale Achse 35, 25 in Figur 9A zeigt jedoch eine Abweichung an denjenigen Positionen, an denen die Transducer 300, 200 und 320, 220 positioniert sind. Typischerweise bewirkt das Material der Transducer eine Verschiebung der neutralen Achse, welche zu den Transducern hin gerichtet ist. Dadurch wird eine gegenüber dem restlichen Schichtaufbau vergleichsweise verringerte Längenänderung an den Positionen der Transducer bei Verbiegung oder Veränderung der Oberflächenspannung erzeugt, wodurch die Sensitivität des Sensors verringert ist.

Um diesem unerwünschten Effekt entgegen zu steuern ist in Figur 9B entsprechend einer bevorzugten Ausführungsform eine Funktionsschicht 390, 290 auf die Wandlerschicht 340, 240 aufgebracht. Durch das Aufbringen der Funktionsschicht 390, 290 kann die neutrale Achse 35, 25 in der Schichtstruktur verschoben werden.

Dies ist in Figur 9B gezeigt. Verglichen mit der neutralen Achse 35, 25 aus Figur 9A ist die neutrale Achse in Figur 9B nach unten verschoben. Um die neutrale Achse 36, 25 durch diese zusätzliche Funktionsschicht 340, 240 nach unten zu verschieben, muss gemäß dieser Ausführungsform der Elastizitätsmodul der Funktionsschicht 340, 240 deutlich kleiner als der Elastizitätsmodul der darunterliegenden Schichtstruktur sein. Weiterhin muss die Dicke der Funktionsschicht 340, 240 ausreichend klein sein, um die gezeigte Verschiebung der neutralen Achse 35, 25, welche von den Tranducern weg gerichtet ist, zu induzieren.

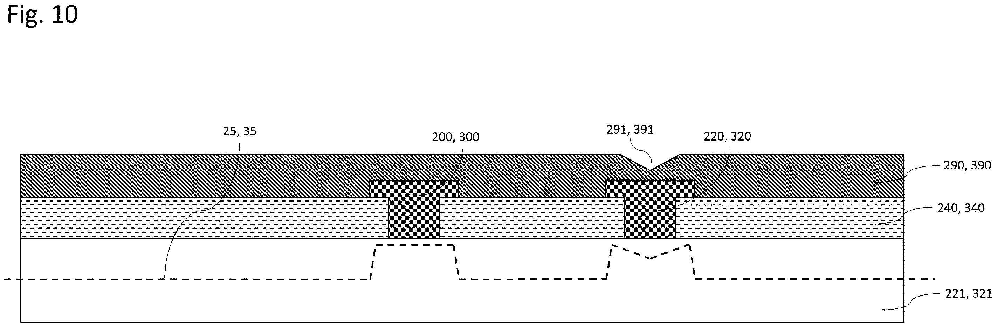

Figur 10 zeigt im Wesentlichen dieselbe Anordnung wie Figur 9B, jedoch ist hier schematisch eine Strukturierung des Höhenprofils der Funktionsschicht 390, 290 gezeigt, welche exemplarisch als Aussparung 391, 291 am Ort des aktiven Referenz- bzw. Testtransducers 320, 220 dargestellt ist. Wie in Figur 10 gezeigt, kann durch die Veränderung des Höhenprofils der Funktionsschicht 390, 290 der Verlauf der neutralen Achse 35, 25 derart beeinflusst werden, dass sich die neutrale Achse am Ort eines Transducers von dem Transducer entfernt.

Durch die Aussparung 391, 291 wird eine gezielte Beeinflussung der Spannungsverteilung des Cantilevers 3, 2 erreicht, welche bevorzugt so gestaltet ist, dass die Dehnung unter einer Verformung des verformbaren Teils 32, 22 des Cantilevers 3, 2 am Ort des aktiven Referenz- bzw. Testtranducers 320, 220 maximal ist.

Es sei darauf hingewiesen, dass es möglich ist, auch auf der strukturierten Funktionsschicht 390, 290 eine Rezeptorschicht 34, 24 aufzubringen.

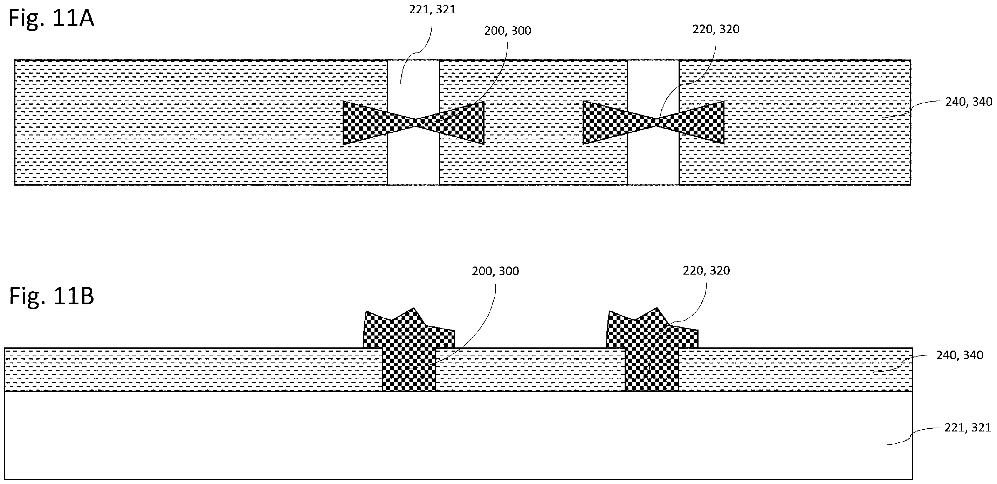

Figur 11A zeigt eine Draufsicht auf einen Cantilever, insbesondere auf die Trägerschicht 321, 221 und auf die darüberliegende Wandlerschicht 340, 240, in welche die passiven Transducer 300, 200 und die aktiven Transducer 320, 220 eingebettet sind.

Beispielsweise kann die Wandlerschicht 340, 240 aus Gold bestehen oder Gold umfassen und Elektroden, also eine elektrische Kontaktierung für die Transducer, umfassen. Wie in der Figur 11 gezeigt, sind die Transducer in eine nicht-trivialen Form strukturiert. Hierbei würde eine triviale Form typischerweise einer rechteckigen Form entsprechen. Durch die Strukturierung kann eine nicht-homogene Spannungsverteilung in den Transducern erreicht werden.

Figur 11B zeigt eine Cantilever Schichtstruktur umfassen eine Trägerschicht 321, 221 und eine Wandlerschicht 340, 240, wobei die passiven Transducer 300, 200 und die aktiven Transducer 320, 220 eine bezüglich ihrem Höhenprofil nicht-triviale Form aufweisen. Hierbei entspräche eine triviale Form einem flachen Höhenprofil der Transducer. Eine solche Strukturierung des Höhenprofils der Transducer kann eine gezielte Beeinflussung der Spannungsverteilung und eine Beeinflussung der neutralen Achse am Ort der Transducer selbst herbeiführen. Hierbei kann die Strukturierung derart gestaltet sein, dass die Spannungsverteilung am jeweiligen Ort der Transducer optimiert wird, also insbesondere die Längenänderung bei Verbiegung der Cantilever am Ort der Transducer maximiert wird.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

Bezugszeichenliste

1     Sensor
10    Biegekante

| | |
|---|---|
| 2 | Testcantilever |
| 20 | Basis |
| 201 | Grundkörper |
| 21 | Biegekante |
| 200 | passiver Testtransducer |
| 22 | verformbarer Teil |
| 221 | Trägerschicht |
| 220 | aktiver Testtransducer |
| 24 | Rezeptorschicht |
| 25 | Neutrale Achse des Testcantilevers |
| 240 | Test-Wandlerschicht |
| 241 | selbstorganisierende Monoschicht |
| 242 | Protein A |
| 243 | Antikörper |
| 244 | Passivierungsschicht |
| 26 | Oberfläche |
| 260 | obere Oberfläche |
| 262 | untere Oberfläche |
| 290 | Test-Funktionsschicht |
| 291 | Aussparung |
| | |
| 3 | Referenzcantilever |
| 30 | Basis |
| 301 | Grundkörper |
| 31 | Biegekante |
| 300 | passiver Referenztransducer |
| 32 | verformbarer Teil |
| 321 | Trägerschicht |
| 320 | aktiver Referenztransducer |
| 34 | Referenzschicht |
| 35 | Neutrale Achse des Referenzcantilevers |
| 340 | Referenz-Wandlerschicht |
| 341 | selbstorganisierende Monoschicht |
| 342 | Protein A |
| 343 | Isotypkontroll-Antikörper |
| 344 | Passivierungsschicht |
| 36 | Oberfläche |
| 360 | obere Oberfläche |
| 362 | untere Oberfläche |
| 390 | Referenz-Funktionsschicht |
| 391 | Aussparung |
| | |
| 4 | Elektronik |
| 40 | Elektrode |
| 400, 401, 402, 403 | Elektroden |
| 42 | Brückenquerspannungsdetektor |
| 44 | AD-Wandler |
| 440 | AD-Wandlerlogik |
| | |
| 5 | Antigen |
| 50 | anderes Antigen |
| | |
| F | Kraft |
| L | Auslenkung |
| D | Dehnung |
| AT | Abstand zwischen aktiven und passiven Transducer |
| AE | Abstand zwischen Elektroden |
| S | Symmetrieachse |
| VB | Brückenquerspannung |

**Patentansprüche**

1. Sensor (1) zur Umwandlung chemischer und/oder biochemischer Information mindestens eines Analyten (90) in einer Probe (9) in ein elektrisches Signal, umfassend

   einen Testcantilever (2), der eine Basis (20) und einen verformbaren Teil (22) aufweist, wobei mindestens auf dem verformbaren Teil (22) eine Rezeptorschicht (24) zur selektiven Aufnahme des Analyten (90) aufgebracht ist, wobei der Testcantilever (2) eine Test-Wandlerschicht (240) aufweist, die auf der Basis (20) einen passiven Testtransducer (200) und auf dem verformbaren Teil (220) einen aktiven Testtransducer (220) umfasst, und wobei zwischen der Test-Wandlerschicht (240) und der Rezeptorschicht (24) eine Test-Funktionsschicht (290) angeordnet ist,
   einen Referenzcantilever (3), der eine Basis (30) und einen verformbaren Teil (32) aufweist, wobei auf dem verformbaren Teil (32) eine Referenzschicht (34) zur selektiven Nichtaufnahme des Analyten (90) aufgebracht ist, wobei der Referenzcantilever (3) eine Referenz-Wandlerschicht (340) aufweist, die auf der Basis (30) einen passiven Referenztransducer (300) und auf dem verformbaren Teil (32) einen aktiven Referenztransducer (320) umfasst, und wobei zwischen der Referenz-Wandlerschicht (340) und der Referenzschicht (34) eine Referenz-Funktionsschicht (390) angeordnet ist.

2. Sensor (1) nach Anspruch 1, **dadurch gekennzeichnet, dass** die aktiven und passiven Referenztransducer (320, 300) und die aktiven und passiven Testtransducer (220, 200) dazu ausgebildet und eingerichtet sind, ein dem Vorkommen und/oder der Konzentration und/oder der Menge des Analyten (90) in der Probe (9) entsprechendes elektrisches Signal auszugeben.

3. Sensor (1) nach Anspruch 1 oder 2, **dadurch gekennzeichnet, dass** die Test-Funktionsschicht (290) den aktiven Testtransducer (220) und/oder den passiven Testtransducer (200) überdeckt und/oder die Referenz-Funktionsschicht (390) den aktiven Referenztransducer (320) und/oder den passiven Referenztransducer (300) überdeckt.

4. Sensor (1) nach einem der vorstehenden Ansprüche, **dadurch gekennzeichnet, dass** die Test-Funktionsschicht (290) eine ebene Oberfläche zur Aufnahme der Rezeptorschicht (24) ausbildet und/oder die Referenz-Funktionsschicht (390) eine ebene Oberfläche zur Aufnahme der Referenzschicht (34) ausbildet.

5. Sensor (1) nach einem der vorstehenden Ansprüche, **dadurch gekennzeichnet, dass** die Test-Funktionsschicht (290) und die Referenz-Funktionsschicht (390) das gleiche Material umfassen oder aus dem gleichen Material sind.

6. Sensor (1) nach einem der vorstehenden Ansprüche, **dadurch gekennzeichnet, dass** das Material der Test-Funktionsschicht (290) und/oder der Referenz-Funktionsschicht (390) zur Anpassung der Oberflächenenergie ausgebildet ist, um die Bindung der Rezeptorschicht (24) an die Test-Funktionsschicht (290) und/oder der Referenzschicht (34) an die Referenz-Funktionsschicht (390) zu erreichen oder zu verbessern.

7. Sensor (1) nach einem der vorstehenden Ansprüche, **dadurch gekennzeichnet, dass** die Test-Funktionsschicht (290) und/oder die Referenz-Funktionsschicht (390) ein elektrisch isolierendes Material mit hohem Isolationswiderstand umfasst oder ist.

8. Sensor (1) nach einem der vorstehenden Ansprüche, **dadurch gekennzeichnet, dass** die Test-Funktionsschicht (290) und/oder die Referenz-Funktionsschicht (390) ein poröses Material umfasst, das eine Teildurchlässigkeit für ausgewählte Stoffe und/oder Analyten ermöglicht.

9. Sensor (1) nach einem der vorstehenden Ansprüche, **dadurch gekennzeichnet, dass** die Test-Wandlerschicht (240) Elektroden zur Kontaktierung des passiven Testtransducers (200) und/oder des aktiven Testtransducers (220) umfasst und/oder die Referenz-Wandlerschicht (340) Elektroden zur Kontaktierung des passiven Referenztransducers (300) und/oder des aktiven Referenztransducers (320) umfasst und die Elektroden von der Test-Funktionsschicht (290) und/oder der Referenz-Funktionsschicht (390) überdeckt sind.

10. Sensor (1) gemäß einer der voranstehenden Ansprüche, **dadurch gekennzeichnet, dass** eine Passivierungsschicht auf der unteren Oberfläche des Testcantilevers (2) und/oder der unteren Oberfläche des Referenzcantilevers (3) angeordnet ist, die dazu eingerichtet ist, eine Adhäsion eines Analyten auf der Unterseite des Testcantilevers (2) und/oder der Referenzcantilevers (3) zu minimieren.

11. Sensor (1) gemäß einer der voranstehenden Ansprüche, **dadurch gekennzeichnet, dass** auf der Unterseite des Testcantilevers (290) und/oder des Referenzcantilevers (390) eine Rezeptorschicht (24) und/oder Referenzschicht (34) zur selektiven Aufnahme eines Analyten angeordnet ist, wobei bevorzugt die durch Bindung eines Analyten an die Rezeptorschicht (24) und/oder Referenzschicht (34) veränderte Oberflächenspannung der oberen Schicht zur Veränderung der Oberflächenspannung der unteren Schicht entgegengesetzt ist.

12. Sensor (1) gemäß einer der voranstehenden Ansprüche, **dadurch gekennzeichnet, dass** die Materialeigenschaften der Test-Funktionsschicht (290) und/oder der Referenz-Funktionsschicht (280), insbesondere deren Dicke und/oder Elastizitätsmodul, so ausgebildet sind, dass eine durch Verformung des verformbaren Teils (22) des Testcantilevers (2) und/oder des verformbaren Teils (32) des Referenzcantilevers (3) induzierte Verformung im Testtransducer (220) und/oder Referenztransducer (320) maximiert wird.

13. Sensor (1) gemäß einer der voranstehenden Ansprüche, **dadurch gekennzeichnet, dass** die Test-Funktionsschicht (290) und/oder der Referenz-Funktionsschicht (390) geometrisch so strukturiert sind, dass eine durch Verformung des verformbaren Teils (22) des Testcantilevers (2) und/oder des verformbaren Teils (32) des Referenzcantilevers (3) induzierte Verformung im Testtransducer (220) und/oder Referenztransducer (320) maximiert wird.

14. Sensor (1) gemäß einer der voranstehenden Ansprüche, **dadurch gekennzeichnet, dass** durch Strukturierung der Test-Transducer (200, 300) und/oder der Referenz-Transducer (220, 320) in der Horizontalebene der Cantilever die Sensitivität der Transducer durch eine Maximierung der Verformung beziehungsweise Längenänderung am Ort der Transducer optimiert wird.

15. Sensor (1) gemäß einer der voranstehenden Ansprüche, **dadurch gekennzeichnet, dass** der Test-Transducer (200, 300) und/oder der Referenz-Transducer (220, 320) geometrisch so strukturiert sind, dass die Sensitivität der Transducer durch eine Maximierung der Verformung beziehungsweise Längenänderung am Ort der Transducer optimiert wird, wobei die räumlich lange Seite des Test-Transducers (200, 300) und/oder die räumlich lange Seite des Referenz-Transducers (220, 320) parallel zur Hauptkomponente der Verformung des verformbaren Teils (22) des Testcantilevers (2) und/oder des verformbaren Teils (32) des Referenzcantilevers (3) ausgerichtet sind.

## Claims

1. Sensor (1) for the conversion of chemical and/or biochemical information from at least one analyte (90) in a sample (9) to an electrical signal, comprising

   a test cantilever (2) having a base (20) and a deformable part (22), with a receptor layer (24) for selective uptake of the analyte (90) applied at least atop the deformable part (22), wherein the test cantilever (2) has a test converter layer (240) comprising a passive test transducer (200) on the base (20) and an active test transducer (220) on the deformable part (220), and wherein a test function layer (290) is arranged between the test converter layer (240) and the receptor layer (24),
   a reference cantilever (3) having a base (30) and a deformable part (32), with a reference layer (34) for selective non-uptake of the analyte (90) applied atop the deformable part (32), wherein the reference cantilever (3) has a reference converter layer (340) comprising a passive reference transducer (300) on the base (30) and an active reference transducer (320) on the deformable part (32), and wherein a reference function layer (390) is arranged between the reference converter layer (340) and the reference layer (34).

2. Sensor (1) according to claim 1, **characterized in that** the active and passive reference transducers (320, 300) and the active and passive test transducers (220, 200) are formed and configured to output an electrical signal corresponding to the occurrence and/or the concentration and/or the amount of the analyte (90) in the sample (9).

3. Sensor (1) according to claim 1 or 2, **characterized in that** the test function layer (290) covers the active test transducer (220) and/or the passive test transducer (200), and/or the reference function layer (390) covers the active reference transducer (320) and/or the passive reference transducer (300).

4. Sensor (1) according to any of the preceding claims, **characterized in that** the test function layer (290) forms a flat surface to accommodate the receptor layer (24) and/or the reference function layer (390) forms a flat surface to accommodate the reference layer (34).

5. Sensor (1) according to any of the preceding claims, **characterized in that** the test function layer (290) and the reference function layer (390) comprise the same material or are composed of the same material.

6. Sensor (1) according to any of preceding claims, **characterized in that** the material of the test function layer (290) and/or of the reference function layer (390) is configured to adjust the surface energy in order to achieve or to improve the binding of the receptor layer (24) to the test function layer (290) and/or of the reference layer (34) to the reference function layer (390).

7. Sensor (1) according to any of the preceding claims, **characterized in that** the test function layer (290) and/or the reference function layer (390) comprises or is an electrically insulating material having high insulation resistance.

8. Sensor (1) according to any of the preceding claims, **characterized in that** the test function layer (290) and/or the reference function layer (390) comprises a porous material that enables partial permeability to selected substances and/or analytes.

9. Sensor (1) according to any of the preceding claims, **characterized in that** the test converter layer (240) comprises electrodes for contacting the passive test transducer (200) and/or the active test transducer (220), and/or the reference converter layer (340) comprises electrodes for contacting the passive reference transducer (300) and/or the active reference transducer (320), and the electrodes are covered by the test function layer (290) and/or the reference function layer (390).

10. Sensor (1) according to any of the preceding claims, **characterized in that** a passivation layer arranged atop the lower surface of the test cantilever (2) and/or the lower surface of the reference cantilever (3) is configured to minimize adhesion of an analyte on the underside of the test cantilever (2) and/or reference cantilever (3).

11. Sensor (1) according to any of the preceding claims, **characterized in that** on the underside of the test cantilever (290) and/or of the reference cantilever (390) a receptor layer (24) and/or reference layer (34) for selective uptake of an analyte is arranged, where preferably the change in surface tension of the upper layer as a result of binding of an analyte to the receptor layer (24) and/or reference layer (34) is the opposite of the change in surface tension of the lower layer.

12. Sensor (1) according to any of the preceding claims, **characterized in that** the material properties of the test function layer (290) and/or the reference function layer (280), in particular the thickness and/or modulus of elasticity, are configured to maximize a deformation in the test transducer (220) and/or reference transducer (320) induced by deformation of the deformable part (22) of the test cantilever (2) and/or of the deformable part (32) of the reference cantilever (3).

13. Sensor (1) according to any of the preceding claims, **characterized in that** the test function layer (290) and/or the reference function layer (390) are geometrically structured so as to maximize a deformation in the test transducer (220) and/or reference transducer (320) induced by deformation of the deformable part (22) of the test cantilever (2) and/or of the deformable part (32) of the reference cantilever (3).

14. Sensor (1) according to any of the preceding claims, **characterized in that** by structuring of the test transducers (200, 300) and/or of the reference transducers (220, 320) in the horizontal plane of the cantilevers the sensitivity of the transducers is optimized by maximization of the deformation or change in length at the site of the transducers.

15. Sensor (1) according to any of the preceding claims, **characterized in that** the test transducer (200, 300) and/or the reference transducer (220, 320) are geometrically structured such that the sensitivity of the transducers is optimized by maximization of the deformation or change in length at the site of the transducers, where the spatially long side of the test transducer (200, 300) and/or the spatially long side of the reference transducer (220, 320) are aligned parallel to the main component of the deformation of the deformable part (22) of the test cantilever (2) and/or of the deformable part (32) of the reference cantilever (3).

**Revendications**

1. Capteur (1) pour la conversion d'informations chimiques et/ou biochimiques d'au moins un analyte (90) dans un échantillon (9) en un signal électrique, comprenant

un cantilever de test (2) qui présente une base (20) et une partie déformable (22), dans lequel une couche de récepteur (24) est appliquée au moins sur la partie déformable (22) pour recevoir sélectivement l'analyte (90), dans lequel le cantilever de test (2) présente une couche de transducteur de test (240) qui comprend un transducteur de test passif (200) sur la base (20) et un transducteur de test actif (220) sur la partie déformable (220), et dans lequel une couche fonctionnelle de test (290) est disposée entre la couche de transducteur de test (240) et la couche de récepteur (24), un cantilever de référence (3) qui présente une base (30) et une partie déformable (32), dans lequel une couche de référence (34) est appliquée sur la partie déformable (32) pour ne pas recevoir sélectivement l'analyte (90), dans lequel le cantilever de référence (3) présente une couche de transducteur de référence (340) qui comprend un transducteur de référence passif (300) sur la base (30) et un transducteur de référence actif (320) sur la partie déformable (32), et dans lequel une couche fonctionnelle de référence (390) est disposée entre la couche de transducteur de référence (340) et la couche de référence (34).

2. Capteur (1) selon la revendication 1, **caractérisé en ce que** les transducteurs de référence actifs et passifs (320, 300) et les transducteurs de test actifs et passifs (220, 200) sont conçus et configurés pour émettre un signal électrique correspondant à la présence et/ou à la concentration et/ou à la quantité de l'analyte (90) dans l'échantillon (9).

3. Capteur (1) selon la revendication 1 ou 2, **caractérisé en ce que** la couche fonctionnelle de test (290) recouvre le transducteur de test actif (220) et/ou le transducteur de test passif (200) et/ou la couche fonctionnelle de référence (390) recouvre le transducteur de référence actif (320) et/ou le transducteur de référence passif (300).

4. Capteur (1) selon l'une quelconque des revendications précédentes, **caractérisé en ce que** la couche fonctionnelle de test (290) forme une surface plane pour recevoir la couche de récepteur (24) et/ou la couche fonctionnelle de référence (390) forme une surface plane pour recevoir la couche de référence (34).

5. Capteur (1) selon l'une quelconque des revendications précédentes, **caractérisé en ce que** la couche fonctionnelle de test (290) et la couche fonctionnelle de référence (390) comprennent le même matériau ou sont du même matériau.

6. Capteur (1) selon l'une quelconque des revendications précédentes, **caractérisé en ce que** le matériau de la couche fonctionnelle de test (290) et/ou de la couche fonctionnelle de référence (390) est conçu pour adapter l'énergie de surface pour obtenir ou améliorer la liaison de la couche de récepteur (24) à la couche fonctionnelle de test (290) et/ou de la couche de référence (34) à la couche fonctionnelle de référence (390).

7. Capteur (1) selon l'une quelconque des revendications précédentes, **caractérisé en ce que** la couche fonctionnelle de test (290) et/ou la couche fonctionnelle de référence (390) comprend ou est un matériau électriquement isolant avec une résistance d'isolement élevée.

8. Capteur (1) selon l'une quelconque des revendications précédentes, **caractérisé en ce que** la couche fonctionnelle de test (290) et/ou la couche fonctionnelle de référence (390) comprend un matériau poreux qui permet une perméabilité partielle à des substances et/ou analytes sélectionnés.

9. Capteur (1) selon l'une quelconque des revendications précédentes, **caractérisé en ce que** la couche de transducteur de test (240) comprend des électrodes pour la mise en contact du transducteur de test passif (200) et/ou du transducteur de test actif (220) et/ou la couche de transducteur de référence (340) comprend des électrodes pour la mise en contact du transducteur de référence passif (300) et/ou du transducteur de référence actif (320) et les électrodes sont recouvertes par la couche fonctionnelle de test (290) et/ou la couche fonctionnelle de référence (390).

10. Capteur (1) selon l'une quelconque des revendications précédentes, **caractérisé en ce qu'**une couche de passivation est disposée sur la surface inférieure du cantilever de test (2) et/ou la surface inférieure du cantilever de référence (3) qui est configurée pour minimiser l'adhérence d'un analyte sur le côté inférieur du cantilever de test (2) et/ou du cantilever de référence (3).

11. Capteur (1) selon l'une quelconque des revendications précédentes, **caractérisé en ce que** une couche de récepteur (24) et/ou une couche de référence (34) est disposée sur le côté inférieur du cantilever de test (290) et/ou du cantilever de référence (390) pour recevoir sélectivement un analyte, dans lequel la tension superficielle de la couche supérieure modifiée par la liaison d'un analyte à la couche de récepteur (24) et/ou à la couche de référence (34) est de préférence opposée à la modification de la tension superficielle de la couche inférieure.

12. Capteur (1) selon l'une quelconque des revendications précédentes, **caractérisé en ce que** les propriétés matérielles de la couche fonctionnelle de test (290) et/ou de la couche fonctionnelle de référence (280), en particulier leur épaisseur et/ou leur module d'élasticité, sont conçues de manière à maximiser une déformation induite par une déformation de la partie déformable (22) du cantilever de test (2) et/ou de la partie déformable (32) du cantilever de référence (3) dans le transducteur de test (220) et/ou le transducteur de référence (320).

13. Capteur (1) selon l'une quelconque des revendications précédentes, **caractérisé en ce que** la couche fonctionnelle de test (290) et/ou la couche fonctionnelle de référence (390) sont structurées géométriquement de manière à maximiser une déformation induite par une déformation de la partie déformable (22) du cantilever de test (2) et/ou de la partie déformable (32) du cantilever de référence (3) dans le transducteur de test (220) et/ou le transducteur de référence (320).

14. Capteur (1) selon l'une quelconque des revendications précédentes, **caractérisé en ce que** la sensibilité des transducteurs est optimisée en maximisant la déformation ou la variation de longueur à l'endroit des transducteurs en structurant les transducteurs de test (200, 300) et/ou les transducteurs de référence (220, 320) dans le plan horizontal des cantilevers.

15. Capteur (1) selon l'une quelconque des revendications précédentes, **caractérisé en ce que** les transducteurs de test (200, 300) et/ou les transducteurs de référence (220, 320) sont structurés géométriquement de sorte que la sensibilité des transducteurs soit optimisée par une maximisation de la déformation ou de la variation de longueur à l'endroit des transducteurs, dans lequel le côté spatialement long des transducteurs de test (200, 300) et/ou le côté spatialement long des transducteurs de référence (220, 320) sont orientés parallèlement au composant principal de la déformation de la partie déformable (22) du cantilever de test (2) et/ou de la partie déformable (32) du cantilever de référence (3).

EP 4 328 578 B1

Fig. 1

Fig. 2A

Fig. 2B

EP 4 328 578 B1

EP 4 328 578 B1

Fig. 2C

22

20

Fig. 2D

Dq

Dl

Fig. 3A

EP 4 328 578 B1

Fig. 3B

Fig. 4

Fig. 5A

EP 4 328 578 B1

Fig. 5B

Fig. 5C

Fig. 6

Fig. 7

EP 4 328 578 B1

Fig. 8

2, 3

20, 30　　　　　21, 31　　　　22, 32

24, 34

290, 390

240, 340

221, 321

200, 300　　　220, 320

201, 301

EP 4 328 578 B1

Fig. 9A

25, 35    200, 300    220, 320

240, 340

221, 321

Fig. 9B

25, 35    200, 300    220, 320

290, 390

240, 340

221, 321

EP 4 328 578 B1

Fig. 10

Fig. 11A

221, 321   200, 300   220, 320

240, 340

Fig. 11B

200, 300   220, 320

240, 340

221, 321

EP 4 328 578 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102020107918 A1
- WO 2007088018 A1
- US 6575020 B1

**In der Beschreibung aufgeführte Nicht-Patentliteratur**

- **RASMUSSEN, P. A.** ; **HANSEN, O.** ; **BOISEN, A.** Cantilever surface stress sensors with single-crystalline silicon piezoresistors. *Applied Physics Letters*, 2005, vol. 86 (20), 203502, https://doi.orq/10.1063/1.1900299